# EUROPEAN PATENT SPECIFICATION

(11) **EP 3 105 345 B1**
(45) Date of publication and mention of the grant of the patent: **10.04.2019**
(21) Application number: 15705264.8
(22) Date of filing: 11.02.2015
(51) Int. Cl.: C12Q 1/6883

(54) **PREDICTING INCREASED RISK FOR ALZHEIMER'S DISEASE**
PROGNOSE EINES ERHÖHTEN RISIKOS FÜR MORBUS ALZHEIMER
PRÉDICTION DE RISQUE ACCRU POUR LA MALADIE D'ALZHEIMER

(30) Priority: 12.02.2014 SE 1450151
(43) Date of publication of application: 21.12.2016
(73) Proprietor: Cray Innovation AB, 756 51 Uppsala (SE)
(72) Inventor: FORSBERG, Lars, 756 51 Uppsala (SE); DUMANSKI, Jan, 756 53 Uppsala (SE)
(74) Representative: Dehns
(86) International application number: PCT/EP2015/052898
(87) International publication number: WO 2015/121317

(56) References cited:
- EP-A1- 0 580 739
- IOUROV I Y ET AL: "Aneuploidy in the normal, Alzheimer's disease and ataxia-telangiectasia brain: Differential expression and pathological meaning", NEUROBIOLOGY OF DISEASE, BLACKWELL SCIENTIFIC PUBLICATIONS, OXFORD, GB, vol. 34, no. 2, 1 May 2009 (2009-05-01), pages 212-220, XP026052955, ISSN: 0969-9961, DOI: 10.1016/J.NBD.2009.01.003 [retrieved on 2009-01-21]
- ANNE E WIKTOR ET AL: "The significance of isolated Y chromosome loss in bone marrow metaphase cells from males over age 50 years", LEUKEMIA RESEARCH, NEW YORK,NY, US, vol. 35, no. 10, 3 May 2011 (2011-05-03), pages 1297-1300, XP028276419, ISSN: 0145-2126, DOI: 10.1016/J.LEUKRES.2011.05.002 [retrieved on 2011-05-09] cited in the application
- P A JACOBS ET AL: "Male breast cancer, age and sex chromosome aneuploidy", BRITISH JOURNAL OF CANCER, vol. 108, no. 4, 8 January 2013 (2013-01-08), pages 959-963, XP055170899, GB ISSN: 0007-0920, DOI: 10.1038/bjc.2012.577 cited in the application
- ROBBERECHT C ET AL: "Piecing together the problems in diagnosing low-level chromosomal mosaicism", GENOME MEDICINE, vol. 2, no. 47, 1 January 2010 (2010-01-01), pages 1-3, XP055181091,
- KINGSBURY M A ET AL: "Aneuploidy in the normal and diseased brain", CMLS CELLULAR AND MOLECULAR LIFE SCIENCES, BIRKHÄUSER-VERLAG, BA, vol. 63, no. 22, 4 September 2006 (2006-09-04), pages 2626-2641, XP019457682, ISSN: 1420-9071, DOI: 10.1007/S00018-006-6169-5
- YUROV YURI B ET AL: "GIN'n'CIN hypothesis of brain aging: deciphering the role of somatic genetic instabilities and neural aneuploidy during ontogeny", MOLECULAR CYTOGENETICS, BIOMED CENTRAL LTD, LONDON UK, vol. 2, no. 1, 25 November 2009 (2009-11-25), page 23, XP021064833, ISSN: 1755-8166, DOI: 10.1186/1755-8166-2-23
- L. A. FORSBERG ET AL: "Non-heritable genetics of human disease: spotlight on post-zygotic genetic variation acquired during lifetime", JOURNAL OF MEDICAL GENETICS, vol. 50, no. 1, 21 November 2012 (2012-11-21), pages 1-10, XP055181034, ISSN: 0022-2593, DOI: 10.1136/jmedgenet-2012-101322 cited in the application
- FAGGIOLI F ET AL: "Chromosome-specific accumulation of aneuploidy in the aging mouse brain", HUMAN MOLECULAR GENETICS, vol. 21, no. 24, 6 September 2012 (2012-09-06), pages 5246-5253, XP055405254, ISSN: 0964-6906, DOI: 10.1093/hmg/dds375 -& FAGGIOLI F ET AL: "Supplementary Materials - Chromosome-specific accumulation of aneuploidy in the aging mouse brain", HUMAN MOLECULAR GENETICS, vol. 21, no. 24, 15 December 2012 (2012-12-15), pages 5246-5253, XP055405279, ISSN: 0964-6906, DOI: 10.1093/hmg/dds375

## Description

### TECHNICAL FIELD

The present embodiments generally relate to predicting increased risk of developing Alzheimer's disease among male subjects using a genetic marker.

### BACKGROUND

There currently exist many methods for prediction of risks for various diseases. An example might be an increased blood pressure, which is associated with higher risk of stroke and cardiovascular disease. Other examples related to cancer are screening using breast mammography for detection of breast cancer and radiology of the lung for detection of asymptomatic lung cancers. However, the latter two methods detect disease processes that have already progressed to radiologically detectable tumors.

The field of human genetics has also generated a number of methods, which are useful in early prediction of risk for various diseases and these methods can be broadly divided into three categories. Cytogenetic analyses were the first allowing discovery of human mutations (changes on a chromosomal level or large sub-chromosomal aberrations), which allowed calculation of the risk of development of a specific disease phenotype, usually in the context of prenatal diagnostics. For instance, trisomy 21, which is connected with a strong risk for the development of Down's syndrome, is a classic example. This and other various cytogenetically detectable chromosomal aberrations usually originate from errors occurring in meiosis of one of the parents and lead to aberrant phenotype in developing embryos or children.

The second large genetic field is concerning monogenic disorders. During the last two decades of the past century, the field of human genetics was to a large extent dominated by studies of monogenic disorders, leading to cloning of a very large number of genes implicated in disease development. Mutations in the genes causing monogenic disorders were usually either inherited from an affected parent or were occurring as new mutations during meiosis leading to a gamete carrying a disease mutation. The importance of these methods is also mainly in the context of prenatal diagnostics and the general medical impact is limited due to the fact that the majority of monogenic disorders are usually rare. A few relevant examples might be Huntington's disease and familial breast or colon cancer.

In addition, the completion of sequencing of the human genome and discovery of a frequent inter-individual variation in DNA sequence for single nucleotides, so called single nucleotide polymorphisms (SNPs), have opened another field of investigations in human genetics. These analyses are usually abbreviated as Genome Wide Association Studies (GWAS) and are based on an assumption that nucleotide variants connected with a certain disease risk are either inherited from a parent or they occur very early during embryonic development and are present in DNA of all cells in the body. A very large number of such studies have been published during the past 10 years, dealing with a wide variety of human phenotypes and complex common disorders. However, the predictions of risks associated between nucleotide variants and phenotypes are usually very small and the portion of the estimated disease heritability explained by the GWAS findings has also been unexpectedly low [1]. For instance, in the field of Alzheimer's disease, numerous GWAS studies have been performed and provided a modest amount of new information. In the latest of these attempts, 19 susceptibility loci for late-onset Alzheimer's disease (LOAD) were identified. However, a major portion of the genetic risk for this disease remains unexplained [2].

Thus, in summary for genetic methods of disease risk predictions, during the past decades projects in human genetics investigating correlations between genotype and phenotype have mostly focused on analyses of the inherited genome, which is composed of the variants of genes that were transferred from the parents to the offspring. The prevailing approach has been analysis of DNA from a single tissue (usually blood) that was sampled at a single time point (non-longitudinal sampling). The rationale in these studies has been the assumption that the vast majority of the cells in the human soma are genetically identical; in other words that the genome of somatic cells is stable across the human life span.

Chromosome Y is the human male sex chromosome. It has been known for more than half a century, first published in 1963, that elderly males frequently lose chromosome Y in normal hematopoietic cells [3, 4]. However, the clinical consequences of this aneuploidy have been unclear and the currently prevailing consensus in the literature suggests that this genetic change should be considered phenotypically neutral and related to normal aging [5-1 0, 24].

Alzheimer's disease (AD) is a major cause of morbidity and mortality among late adult/elderly/old humans in developed countries. The clinical diagnosis of AD is usually made when a patient shows symptoms. Early symptoms are often mistakenly thought to be 'age-related' concerns, or manifestations of stress [11]. In the early stages, the most common symptom is difficulty in remembering recent events. When AD is suspected, the diagnosis is usually confirmed with tests that evaluate behavior and thinking abilities, often followed by a brain scan if available.

The pathophysiology of Alzheimer's disease is characterised by loss of neurons and synapses in the cerebral cortex and certain subcortical regions which results in gross atrophy of the affected regions. MRI and PET studies have documented reductions in the size of specific brain regions as patients progressed from mild cognitive impairment to Alzheimer's disease, and also in comparison with similar images from healthy older adults.

Amyloid plaques and neurofibrillary tangles are visible by microscopy in brains of those with AD. Plaques are dense, mostly insoluble deposits of beta-amyloid peptide and cellular material and are found outside and around neurons whilst neurofibrillary tangles are aggregates of hyperphosphyorylated microtubule-associated protein tau and accumulate inside cells. Although many older individuals develop some plaques and tangles as a consequence of ageing, the brains of people with AD have a greater number of them in specific brain regions such as the temporal lobe.

As the disease advances, symptoms can include confusion, irritability, aggression, mood swings, trouble with language, and long-term memory loss. As the sufferer declines they often withdraw from family and society. Gradually, bodily functions are lost, ultimately leading to death [11]. Current treatments only help with the symptoms of the disease and there are no available treatments that stop or reverse the progression of the disease. In developed countries, AD is one of the most costly diseases to society [12, 13].

### SUMMARY

It is a general objective to provide a tool that can be used to identify subjects having a risk of developing Alzheimer's disease.

It is a particular objective to provide a tool that can be used to identify subjects currently not diagnosed with Alzheimer's disease but having an increased risk of developing Alzheimer's disease in the future.

These and other objectives are met by embodiments as defined herein.

An aspect of the embodiments relates to a method of predicting whether a male subject has an increased risk of developing Alzheimer's disease. The method comprises determining a fraction of cells, from a biological sample obtained from the male subject, which have lost chromosome Y. The determined fraction of cells is compared to a predefined threshold. The method also comprises predicting whether the male subject has an increased risk of developing Alzheimer's disease based on the comparison between the fraction of cells and the predefined threshold.

Another aspect of the embodiments relates to the use of loss of chromosome Y (LOY) in cells from a biological sample obtained from a male subject as a genetic marker to predict whether the male subject has an increased risk of developing Alzheimer's disease.

A further aspect of the disclosure relates to a kit for predicting whether a male subject has an increased risk of developing Alzheimer's disease. The kit comprises means for determining a fraction of cells, from a biological sample obtained from the male subject, which have lost chromosome Y. The kit also comprises instructions for comparing the determined fraction to a threshold defined by the instructions. The kit further comprises instructions for predicting whether the male subject has increased risk of developing Alzheimer's disease based on the comparison between the determined fraction and the threshold.

An important feature of the present invention to assess risk of AD is that LOY is determined in normal, healthy cells, not in diseased cells, or cells taken from diseased or unhealthy tissue. Indeed, in the methods of the invention, the cells need not be cells or from tissues which may become diseased. Thus the cells need not be brain cells, and indeed in preferred embodiments are not. Accordingly the invention is not based on determining, measuring or assessing the pathophysiological features of established Alzheimer's disease including cerebral atrophy, beta-amyloid plaques or neurofibrillary tangles as described above.

### BRIEF DESCRIPTION OF THE DRAWINGS

The embodiments, together with further objects and advantages thereof, may best be understood by making reference to the following description taken together with the accompanying drawings, in which:

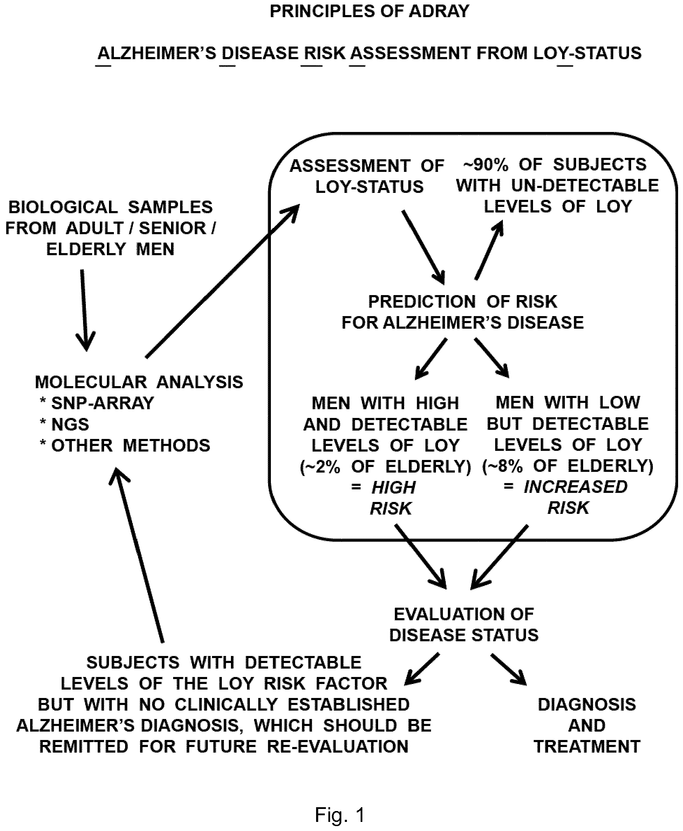

**Fig. 1** - Principles of ADRAY-evaluation (Alzheimer's disease Risk Assessment from LOY-status). This schematic view illustrates the clinical setting and how the ADRAY-evaluation can identify subjects at risk of developing Alzheimer's disease. Black box represents an embodiment, with assessment of the degree of loss of chromosome Y (LOY-status) and subsequent prediction of Alzheimer's disease risk in adult/senior/elderly men. In an embodiment, the Illumina SNP-array technology (Single-Nucleotide-Polymorphism-array) is the molecular analysis performed to produce the data that is analyzed in the ADRAY-evaluation. In other embodiments, any other genetic (or other) methods such as NGS (Next-Generation-Sequencing), CGH-arrays (Comparative-Genomic-Hybridization), PCR-based techniques, RNA-assays, gene-expression technology, methylome technology, protein-assays, cytogenetic and microscopy methods etc. could be used to produce the data that are used to estimate the LOY-status and/or perform the ADRAY-evaluation.

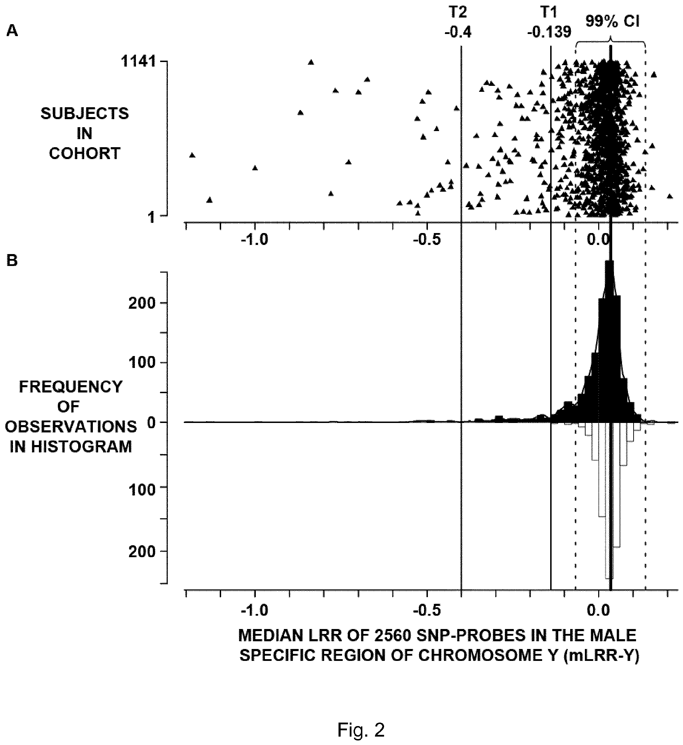

**Fig. 2** - Estimation of frequency of somatic loss of chromosome Y (LOY) in 1141 elderly men of the ULSAM-cohort. LOY frequency estimation was performed after accounting for experimental variation. Panel A shows the median Log R Ratio (LRR) from SNP-array genotyping in the male specific part of chromosome Y (mLRR-Y) and each triangle represents one participant. Panel B shows the distribution of the mLRR-Y (black bars) and the experimental noise (white bars) that were used to find the threshold for estimation of LOY frequency. The dotted black lines represent the 99 % confidence intervals (CI) of the distribution of expected experimental background noise (white bars). Among the analyzed men we found that 14.7 % had a lower median LRR than the lower 99 % CI representing LOY in ∼13.1 % of cells. For estimating the frequency of LOY, we used the lowest value in the noise-distribution as threshold T1 (line at -0.139), which yielded a frequency in the population at 8.2 % of men. The threshold T2 at -0.4 was used in survival analyses.

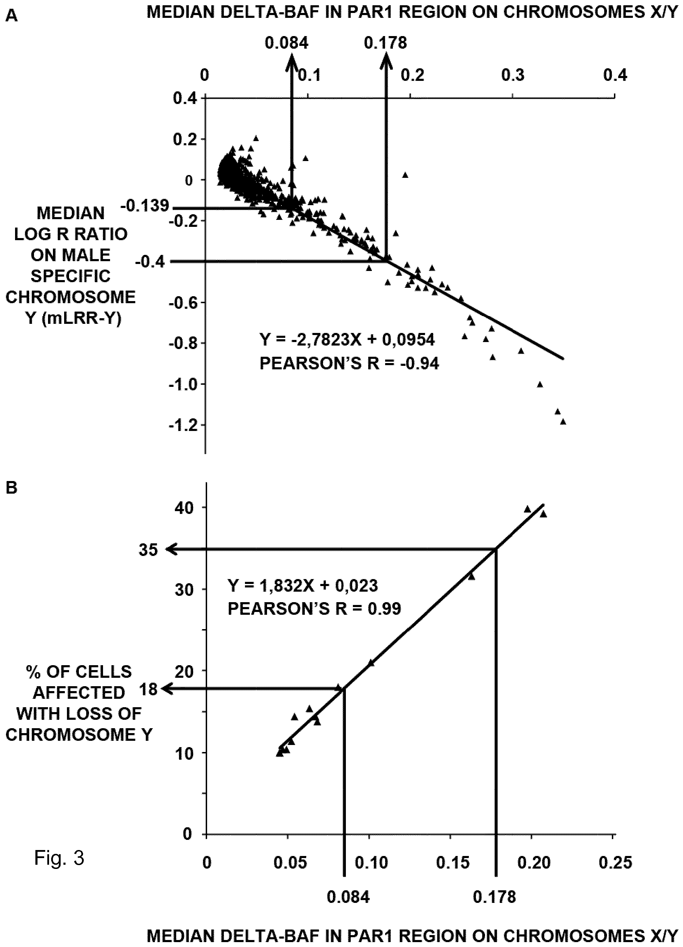

**Fig. 3** - Estimation of the percentage of blood cells affected with loss of chromosome Y (LOY) through analysis of SNP-array data from the pseudoautosomal region 1 (PAR1) of chromosomes X/Y using MAD-software [14]. PAR1 is the largest of the PARs (regions with homologous sequences on chromosomes X and Y) with coordinates 10001-2649520 on Y and 60001-2699520 on X. MAD-software is a tool for detection and quantification of somatic structural variants from SNP-array data, which uses diploid B-allele frequency (BAF) for identification and Log R Ratio (LRR) for quantification of somatic variants and is not originally intended for analyses of chromosome Y data. However, by using the correlation between the LRR in the PAR1-region of Y and the delta-BAF, i.e. the absolute deviation from the expected BAF-value of 0.5 in heterozygous probes, of the PAR1-region of X/Y (panel A), we could use the MAD-quantification of the diploid PAR1 region on chromosomes X/Y to calculate the percentage of cells affected with LOY (panel B). For example, the delta-BAF-value at the LRR-threshold for survival analyses (mLRR-Y ≤ -0.4) was found using the equation given in panel A, i.e. 0.178. This equation (y = -2.7823x + 0.0954) is describing the relationship between mLRR-Y on Y and delta-BAF on X/Y. Next, the percentage of cells affected by LOY was found by applying the equation in panel B that describes the relationship between delta-BAF and the percentage of cells as estimated by the MAD software for 14 cases (y = 1.832x + 0.023). For this example, the delta-BAF of 0.178 translates to LOY in 35 % of cells.

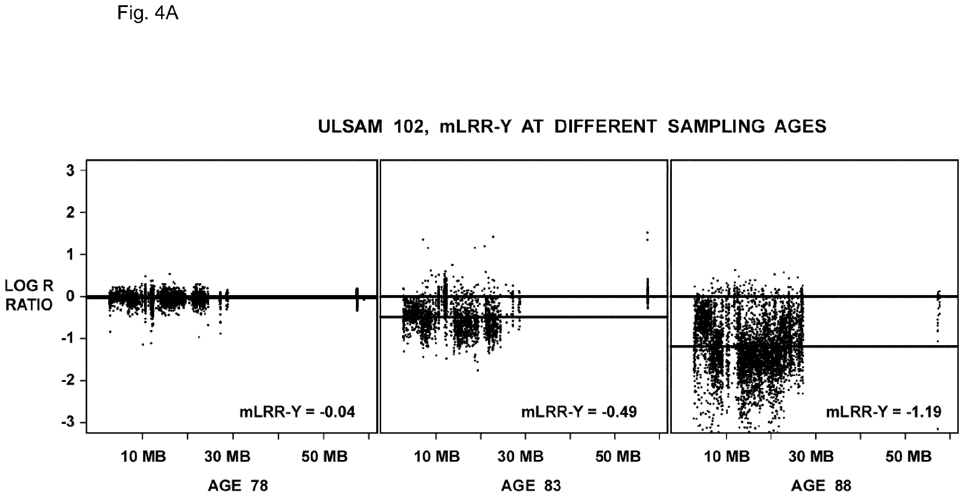

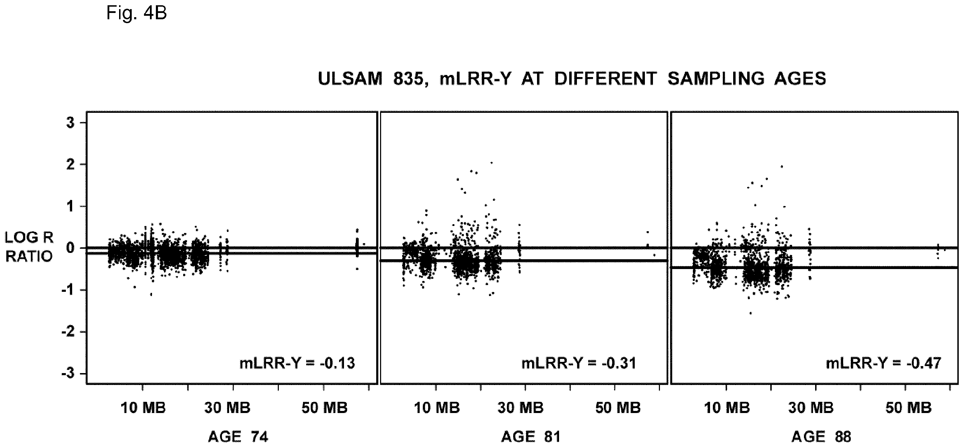

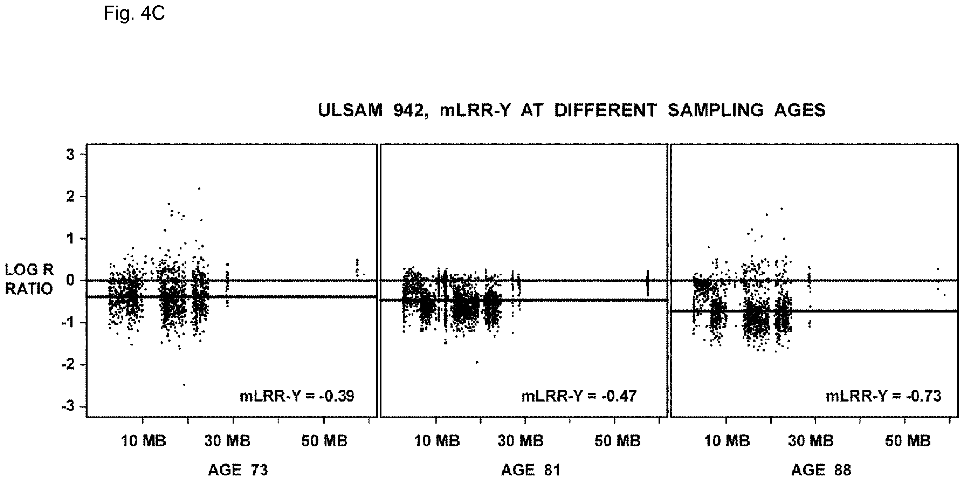

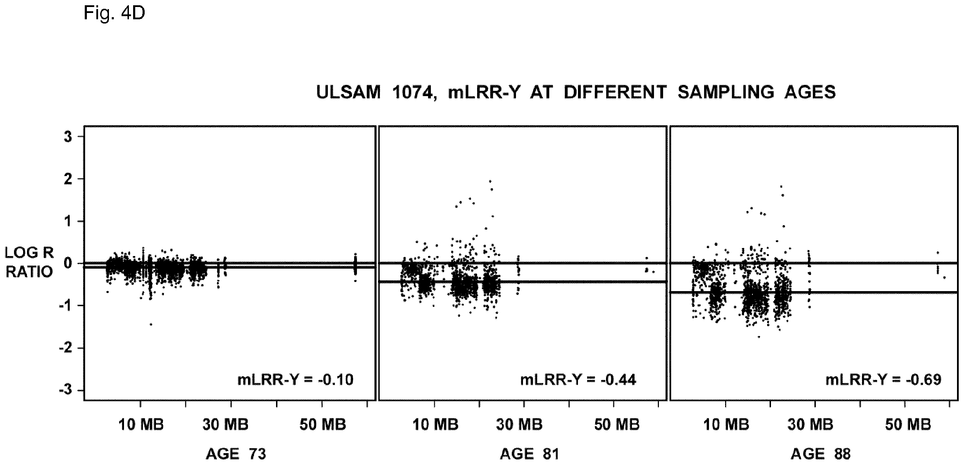

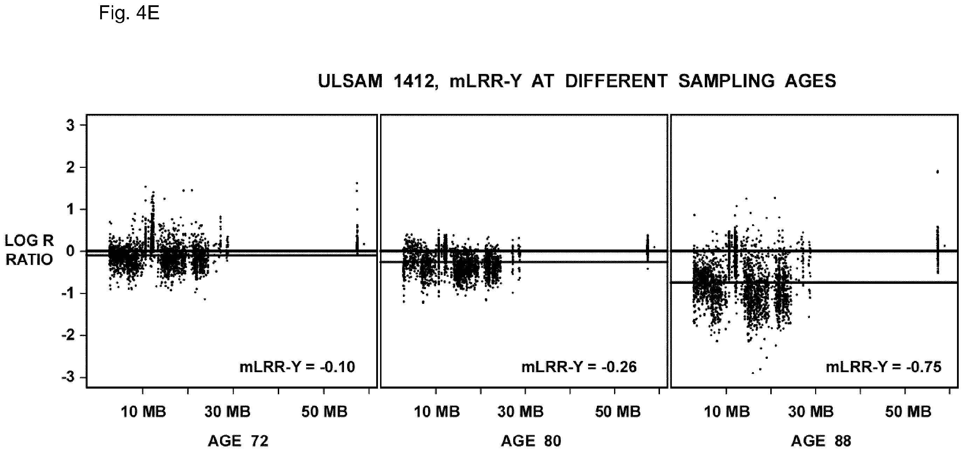

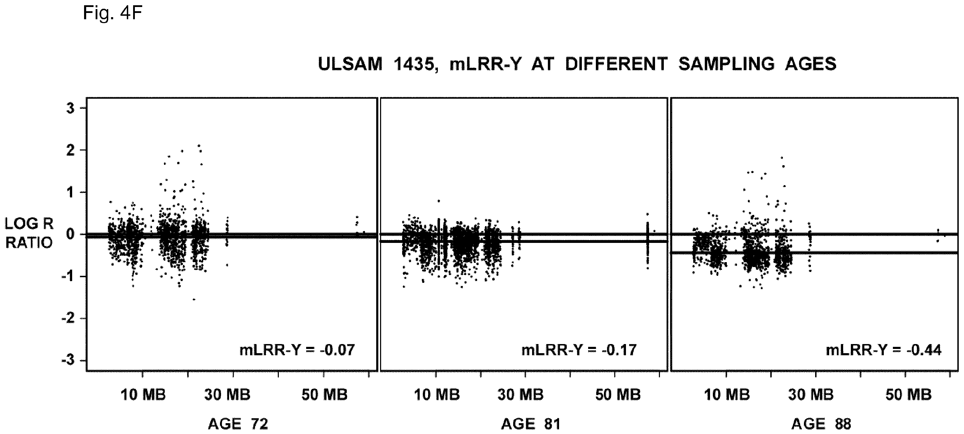

**Fig. 4** - Illustration of longitudinal changes in six ULSAM-participants in panels A-F. For each subject, the Log R Ratio from SNP-array analyses of chromosome Y is displayed from three different ages. The normal state with no (or un-detectable) loss of chromosome Y (LOY) is found close to zero. For example, in panel A, the subject ULSAM 102 at 78 years old shows a normal profile. Subsequently, at ages 83 and 88, the fraction of cells with LOY increases in the blood. A line to indicate the median Log R Ratio of all SNP-probes in the male specific part of chromosome Y (mLRR-Y) is plotted to indicate the level of LOY. Panels B through F show similar patterns of progressive accumulation of cells containing LOY with increasing age in subjects 835, 942, 1074, 1412 and 1435, respectively. These six cases sampled and analyzed in longitudinal fashion shows a typical pattern with a clear increase of the fraction of cells with LOY in blood. This is a strong indication that the affected cells have a proliferative advantage as compared to wildtype cells with unaffected LOY-status.

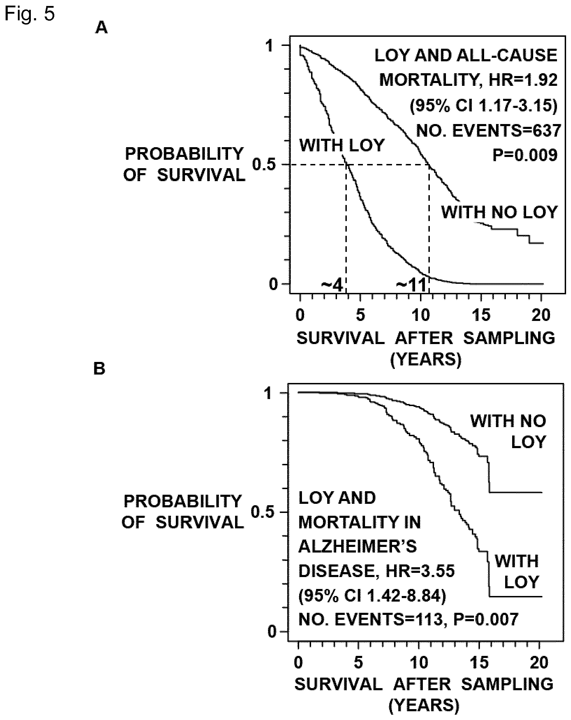

**Fig. 5** - LOY and its effect on mortality in the ULSAM cohort. Panels A and B shows the increased risk for all-cause mortality and increased risk for mortality in AD in men with LOY, respectively. Analysis of all-cause mortality in panel A was based on data from 1153 men and AD mortality in panel B was based on data from 1135 subjects after exclusion of 18 subjects from the AD-analysis since they were diagnosed with AD before genotyping. Hazard ratios (HR), 95 % confidence intervals (CI), number of events and p-values are shown for both models. Results are derived from Cox proportional hazards regression models, with subjects classified into groups 1 and 0 based on their level of LOY. Individuals in the affected group (lower curve in each panel) had LOY in ≥ 35 % of nucleated blood cells (see Fig. 2).

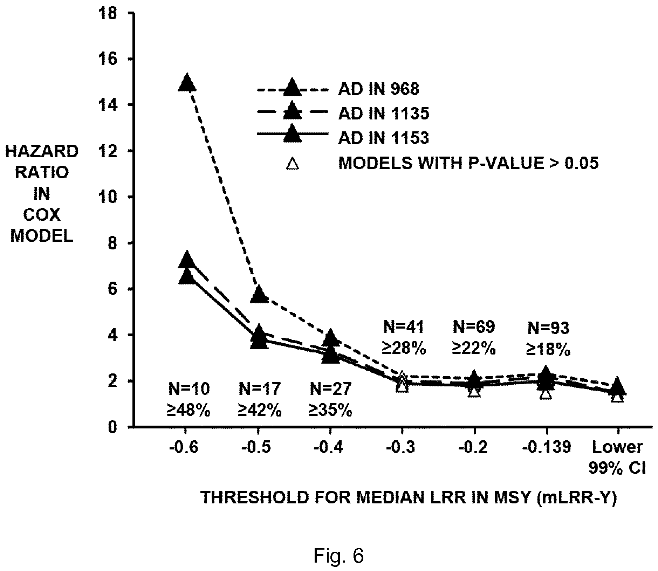

**Fig. 6** - Results from exploratory survival analyses using Cox proportional hazards regression models with different thresholds for classification of participants into groups 1 and 0, based on their level of loss of chromosome Y (LOY) measured as the median Log R Ratio (LRR) in the male specific part of chromosome Y (mLRR-Y). The number of participants (N) with LOY and the minimum percentage of affected cells for each subject are given for each of the tested thresholds. The three curves represent results from analyses of: i) 1153 subjects (the whole ULSAM cohort) with genotyping results that passed quality control criteria; ii) 1135 subjects, which excluded 18 AD patients with diagnosis prior to genotyping; and iii) 968 subjects which excluded subjects diagnosed with AD and any type of cancer prior to genotyping. Exclusion of subjects with Alzheimer and cancer diagnoses improves the predictive power of the Cox-model. Furthermore, exclusion of subjects with cancer diagnoses is motivated by our previous findings linking LOY to risk for cancer. Empty triangles denote models which are not statistically significant.

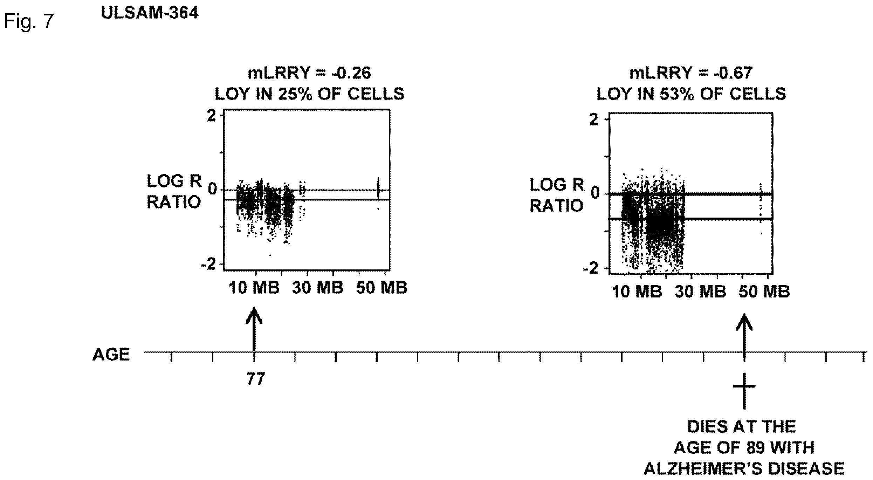

**Fig. 7** - Example 1 shows a patient named ULSAM-364, who was genotyped on SNP-array at two different ages, 77 and 89 years old, and he subsequently died from Alzheimer's disease (AD) at the age of 89. Somatic loss of chromosome Y (LOY) was present at both genotyping ages with 25 % and 53 % of affected cells, respectively. An additional ADRAY-evaluation in between the ages of 77 and 89 would have predicted a risk of AD.

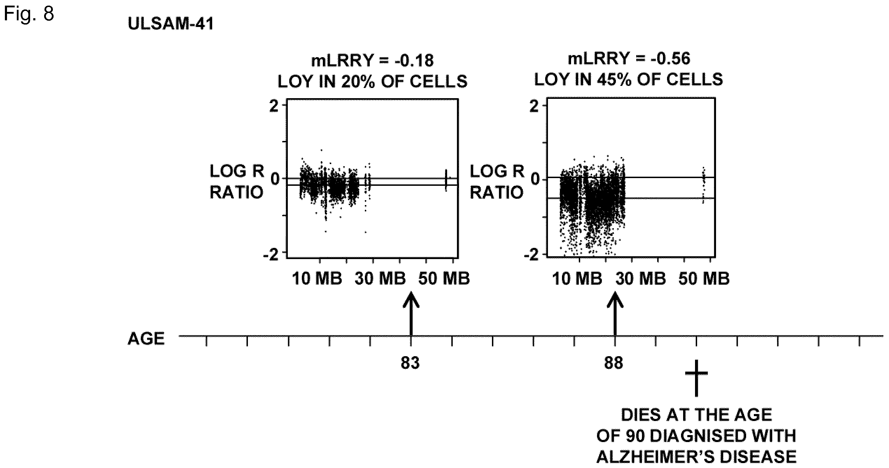

**Fig. 8** - Example 2 shows a subject ULSAM-41 who was genotyped using SNP-array at two ages, 83 and 88 years old, and died at the age of 90 having a diagnosis of Alzheimer's disease (AD). Somatic loss of chromosome Y (LOY) was present at both genotyping ages with 20 % and 45 % of affected cells, respectively. An ADRAY-evaluation at the age of 85 or later would have predicted an increased risk of AD.

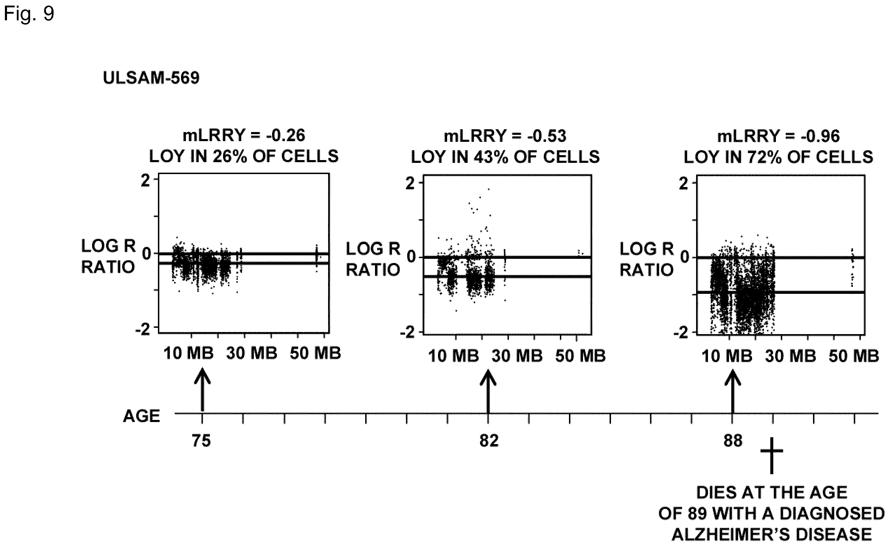

**Fig. 9** - Example 3 showing a subject ULSAM-569 who was genotyped on SNP-arrays at three ages, 75, 82 and 88 years, and died at the age of 89 with a diagnosis of Alzheimer's disease (AD). Somatic loss of chromosome Y (LOY) was present at all genotyping ages with 26 %, 43 % and 72% of affected cells, respectively. An ADRAY-evaluation at the age of 75 years or later would have predicted an increased risk of AD. Screening of blood sampled at even younger ages would probably entail an even earlier risk of future diagnosis of AD.

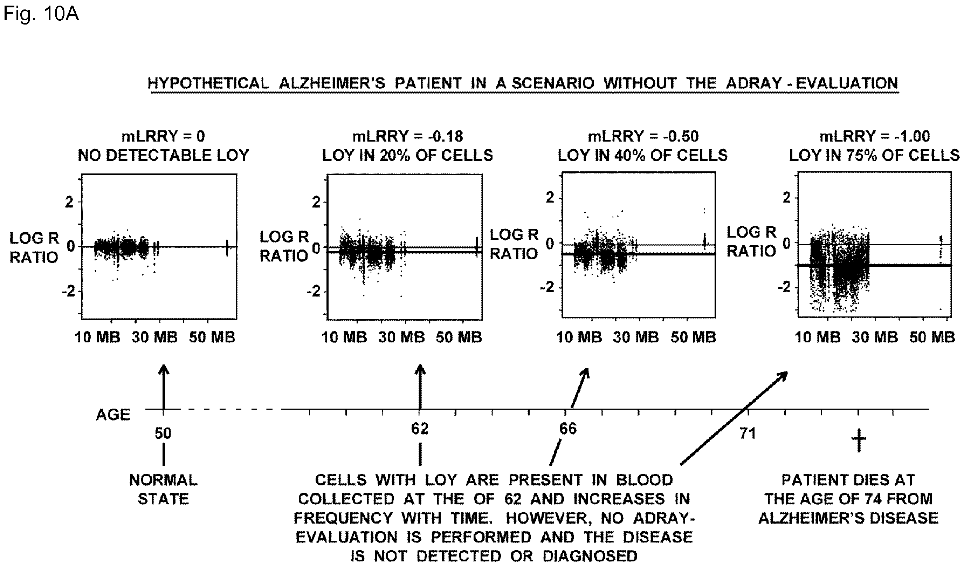

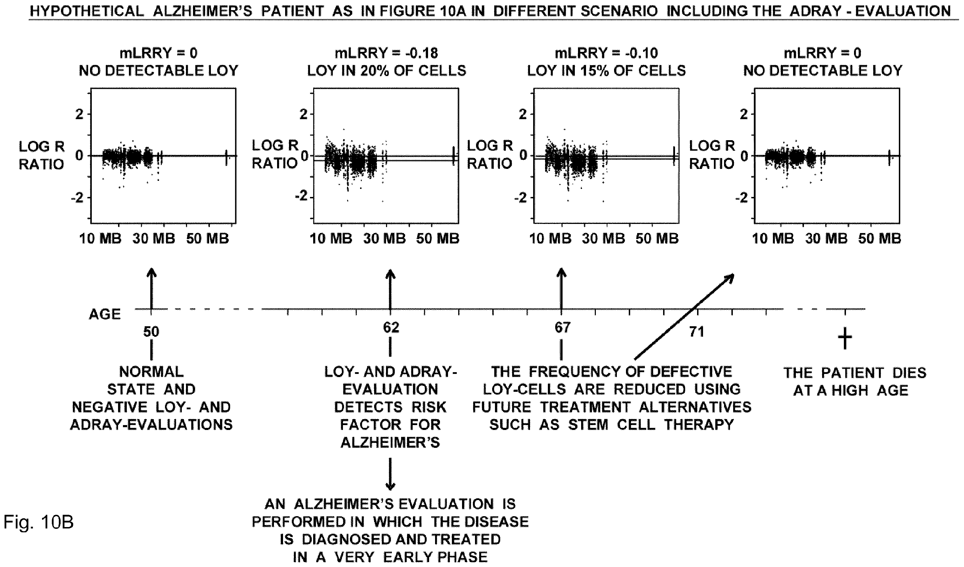

**Fig. 10** - Examples 4 and 5 illustrating the potential benefits of ADRAY-evaluation using two hypothetical patients in two different scenarios, depicted in panels A and B, respectively. In the first scenario shown in panel A, no ADRAY-evaluation is performed and the subject dies from AD at the age of 74. This scenario is very similar to real-life examples 1-3 (shown in in Figs. 7-9). In the alternative scenario shown in panel B, the ADRAY-evaluation performed at the age of 62 detects the risk factor for AD. In this hypothetical patient, future treatment eliminates the damaging effect of cells with LOY. Possible therapy may involve stem cell therapy meant to replace the defective blood clones affected with LOY.

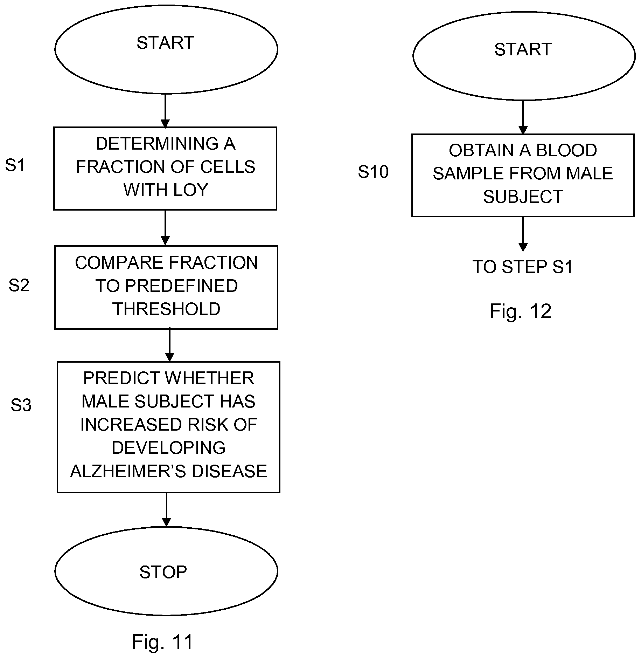

**Fig. 11** - A flow diagram illustrating method of predicting whether a male subject has an increased risk of developing Alzheimer's disease according to an embodiment.
**Fig. 12** - A flow diagram illustrating an additional, optional step of the method as shown in Fig. 11.

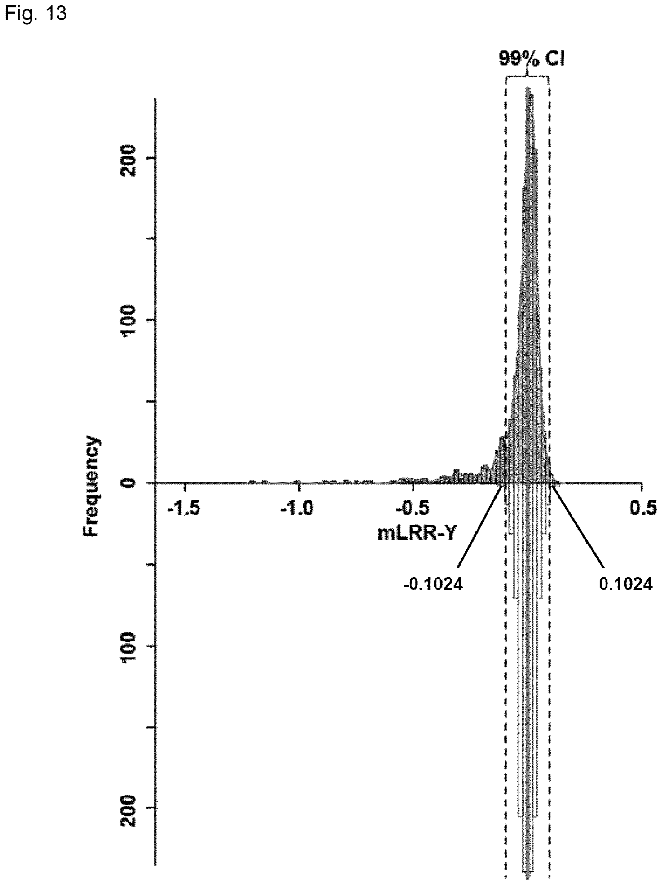

**Fig. 13** - A histogram showing estimation of LOY-frequency in the ULSAM from SNP-array data after accounting for the experimentally induced variation (white bars), as previously described (18). The left tail of the mLRR-Y distribution represents the men that had mLRR-Y values below the lower 99% confidence interval of the experimental noise distribution (-0.1024) and scored with LOY. This represents 12.6% of the cohort.

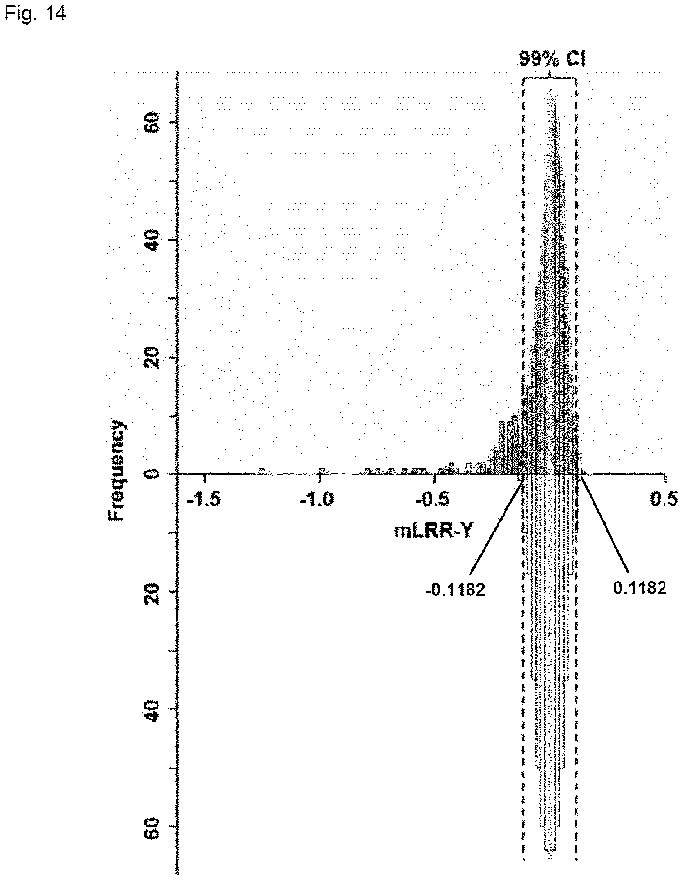

**Fig. 14** - A histogram showing estimation of LOY-frequency in the PIVUS from SNP-array data after accounting for the experimentally induced variation (white bars), as previously described (18). The left tail of the mLRR-Y distribution represents the men that had mLRR-Y values below the lower 99% confidence interval of the experimental noise distribution (-0.1182) and scored with LOY. This represents 15.6% of the cohort.

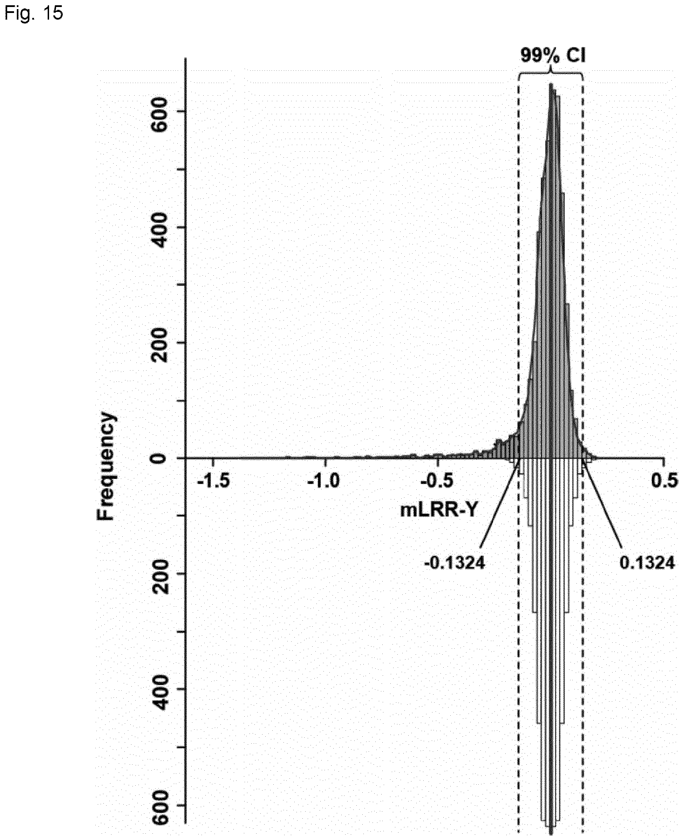

**Fig. 15** - A histogram showing estimation of LOY-frequency in the TwinGene from SNP-array data after accounting for the experimentally induced variation (white bars), as previously described (18). The left tail of the mLRR-Y distribution represents the men that had mLRR-Y values below the lower 99% confidence interval of the experimental noise distribution (-0.1324) and scored with LOY. This represents 7.5% of the cohort. However, of men older than 70 years in the TwinGene study, 15.4% had LOY, which is similar to the LOY frequency in the other cohorts (ULSAM and PIVUS) at the same age range.

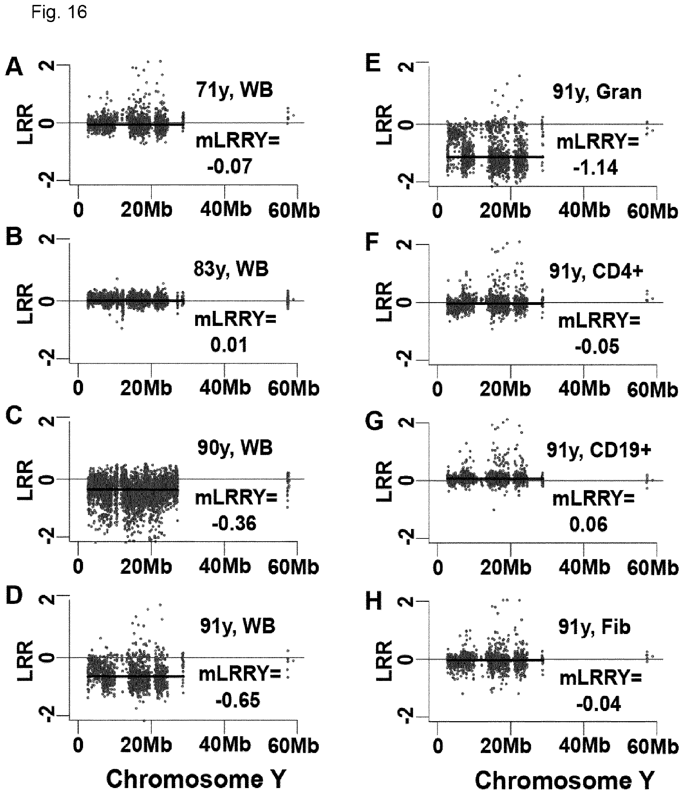

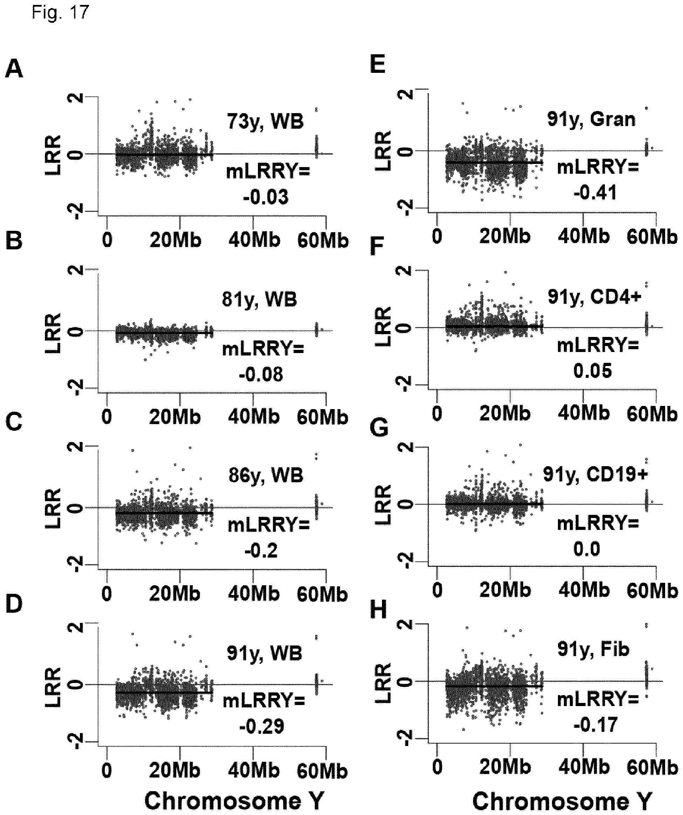

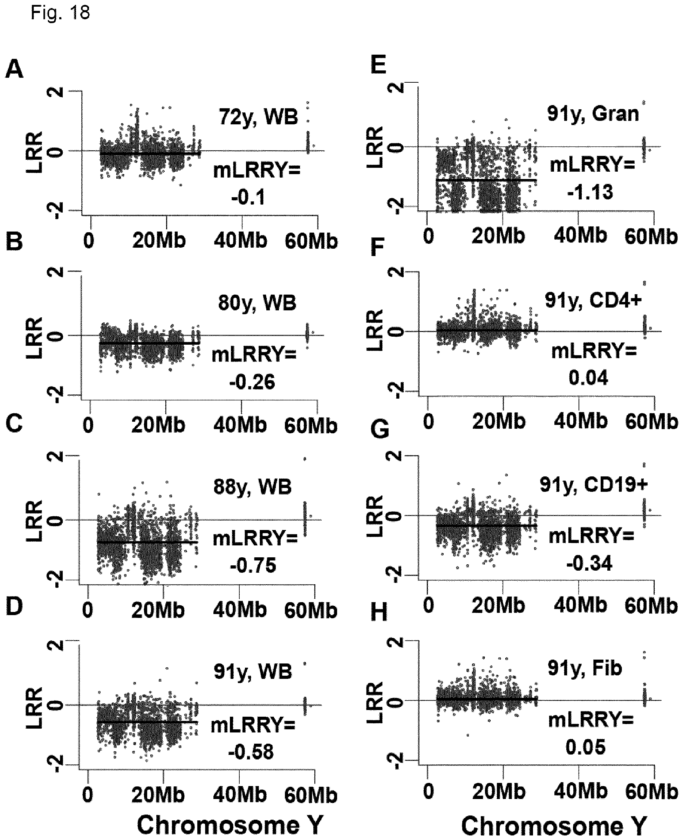

**Fig. 16-18** - Dot plots showing how LOY varies with age and in different cellular compartments of blood in ULSAM subjects 340, 973 and 1412 respectively. Genotyping profile of chromosome Y was performed using Illumina SNP-beadchips, with genotyping of DNA from each individual collected at four different ages during about two decades. In addition to analysis of whole blood, the latest sample was also sorted and blood cells from three major cellular compartments (granulocytes, CD4+ T-lymphocytes and CD19+ B-lymphocytes) was genotyped. Fibroblasts after short-term *in vitro* culture were also analysed. (Abbreviations are as follows: WB, whole blood; Gran, granulocytes; CD4+, T-helper lymphocytes; CD19+, B-lymphocytes; Fib, fibroblasts. Each panel shows LRR (i.e. log R Ratio, dots) for every probe included in the male-specific region of chromosome Y. Thick black lines represent the median LRR values mLRR-Y.)

### DETAILED DESCRIPTION

The present embodiments generally relate to predicting increased risk of developing Alzheimer's disease (AD) among male subjects using a genetic marker.

The present embodiments are related to a field of human genetics generally denoted "somatic mosaicism" or "post-zygotic mosaicism". These terms define the presence of genetically distinct lineages of cells within a single organism, which is derived from the same zygote. In other words, they refer to DNA changes acquired during lifetime, ranging in size from single base pair mutations to aberrations at the chromosomal level [1].

In more detail, the present embodiments are based on the unexpected discovery that loss of human chromosome Y in normal cells, such as nucleated blood cells, of adult and elderly men, is associated with earlier mortality and increased risk for Alzheimer's disease.

In brief, there is, which is further shown herein, a strong association between somatic loss of chromosome Y (LOY) in cells and risk for developing Alzheimer's disease. This opens up for the prediction of individual Alzheimer's disease risks.

Previous studies have described the occurrence of somatic loss of chromosome Y in normal haematopoietic cells, but were unable to describe the link between this aberration and risk for mortality and Alzheimer's disease.

Thus, the present embodiments provide a tool that can be used to test or investigate male subjects that currently are not diagnosed for Alzheimer's disease and are thereby regarded as being healthy at least in terms of not having any diagnosed Alzheimer's disease. The tool is able to identify those healthy individuals that have an increased risk of developing Alzheimer's disease and, consequently, have a risk of suffering from Alzheimer's disease in the future. As described below, the method of the invention has utility in predicting the risk of Alzheimer's disease in male subjects not identified as having Alzheimer's disease. This can include subjects with undetected Alzheimer's disease (that is Alzheimer's disease which is as yet undetected, i.e. undetected at the time of the LOY determination). The method could thus lead to earlier diagnosis of Alzheimer's disease, if the subject is subjected to behavioral and cognitive evaluation or brain scan as a result of the LOY determination and Alzheimer's disease risk prediction. The method may predict an increased risk of Alzheimer's disease in a subject with undetected Alzheimer's disease in early and/or late progression and lead to detection of said disease.

The present embodiments can, thus, be used to screen for and identify male subjects that have an increased risk of developing Alzheimer's disease, compared to male subjects in general, as predicted based on LOY as disclosed herein.

Increased risk should thereby be regarded herein as a risk level of developing Alzheimer's disease that is elevated and thereby higher than any general risk among male subjects of developing Alzheimer's disease. The increased risk can be seen as a risk in addition to and above any normal or baseline risk of developing Alzheimer's disease that exist among all male subjects.

The present embodiments thereby do not provide any Alzheimer's disease diagnosis since the identified male subjects are currently not suffering from any Alzheimer's disease but rather enable identification of those male subjects that have a comparatively high risk of developing Alzheimer's disease during their remaining lifetime.

Increased risk of developing Alzheimer's disease as predicted based on LOY as disclosed herein implies an increased risk of suffering from Alzheimer's disease in the future as compared to control subjects that do not show any significant detectable loss of chromosome Y.

Accordingly, an aspect of the embodiments relates to a method of predicting whether a male (human) subject has an increased risk of developing Alzheimer's disease. The method comprises, as shown in Fig. 11, determining, in step S1, a fraction of cells, from a biological sample obtained from the male subject, that have lost chromosome Y. This fraction is compared to a predefined threshold in step S2. A following step S3 comprises predicting whether the male subject has an increased risk of developing Alzheimer's disease based on the comparison between the fraction and the predefined threshold.

Thus, the embodiments determine the fraction, percentage or degree of cells that have lost chromosome Y in the biological sample taken from the male subject. If this fraction is sufficiently high, as determined in the comparison with the predefined threshold, the male subject is predicted to have an increased risk of developing Alzheimer's disease even if the male subject currently does not have any diagnosed Alzheimer's disease.

In a particular embodiment, determining the fraction in step S1 of Fig. 11 comprises determining a fraction of somatic cells from the biological sample that have lost chromosome Y. Thus, in this embodiment the fraction of cells that have lost Y in the biological sample is determined among somatic cells. This means that any gametes, if present in the biological sample, are preferably excluded from the determination of the fraction of cells.

The present embodiments can generally be used to predict an increased risk of developing Alzheimer's disease. Hence, the embodiments are in particular suitable for predicting whether the male subject has an increased risk of developing Alzheimer's disease based on the comparison between the LOY cell fraction determined in step S1 of Fig. 11 and the predefined threshold.

The biological sample as obtained from the male subject can be any biological sample that comprises cells, preferably somatic cells, of the male subject. Non-limiting but suitable examples of biological sample include a blood sample, a lymph sample, a saliva sample, a urine sample, a feces sample, a cerebrospinal fluid sample, a skin sample, a hair sample or indeed any other tissue sample or type of biopsy material taken from the male subject.

In a particular embodiment, the biological sample is a blood sample. Accordingly, the method as shown in Fig. 11 then preferably comprises an additional step of obtaining a blood sample from the male subject in step S10 as shown in Fig. 12. The determination of the fraction of cells in step S1 of Fig. 11 then preferably comprises determining the fraction of nucleated blood cells from the blood sample that have lost chromosome Y. Thus, in the prediction method of the invention the cells in step S1 may be nucleated blood cells from a blood sample obtained from the male subject.

Accordingly, the cells subjected to the determination in step S1 may be nucleated blood cells from the blood sample, or more generally cells from a biological sample. It is clear from this that the determination in step S1 may be performed on cells from a biological sample, and that this encompasses that for the determination step the cells may be taken or separated from the sample, e.g. prior to the determination being carried out, or that the determination may be performed in a selective manner on a particular set of cells (i.e. a sub-set of cells) in the sample.

A biological sample may thus be subjected to a fractionation or separation step to separate a subset or fraction of cells from the sample for the determination step S1. More particularly, the method of the invention may include a separation or fractionation step of the biological sample prior to carrying out the determination. The determination of the fraction of cells that have lost Y may thus comprise performing the determination on a subset or fraction of cells from the biological sample (i.e. determining the fraction of cells from a subset or fraction of cells from the biological sample that have lost chromosome Y).

Such a separation or fractionation step may be used to separate or isolate a particular cell type or a particular fraction of cells from a biological sample. Accordingly the determination may be performed on a particular cell type or cell fraction from the biological sample.

Methods for fractionating or sorting cells are well known in the art, e.g. for separating a particular cell type from a mixture of cells in a sample such as blood which contains a mixture of different types of cell. This will depend on the nature or type of cell to be separated, but may for example be done by centrifugation or other separation techniques based on the physio-chemical properties of the cell, or by positive and/or negative selection based on cell markers for a particular cell type.

As is well known in the art, a blood sample comprises various blood cells, and in particular red blood cells, i.e. erythrocytes; white blood cells, i.e. leukocytes; and platelets, i.e. thrombocytes.

Generally, red blood cells and platelets lack a cell nucleus in humans. Accordingly, these blood cells are therefore regarded as non-nucleated blood cells. The determination of the fraction of cells in step S1 preferably involves, in this embodiment, determining the fraction of nucleated blood cells that have lost chromosome Y. This means that in preferred embodiment loss of chromosome Y is investigated in white blood cells in the blood sample since the white blood cells, in clear contrast to the red blood cells and the platelets contain a cell nucleus. Thus, step S1 of Fig. 11 then preferably comprises determining the fraction of leukocytes from the blood sample that have lost chromosome Y.

It will be apparent from this that the leukocytes may be separated from the blood sample for the determination step. Thus, the blood sample which is used in the method may be, as well as whole blood, a blood-derived sample, such as (but not limited to) a fraction of leukocytes or PBMC etc. It will be understood that the blood-derived sample will be a sample containing nucleated blood cells.

Accordingly, a blood sample may be sorted or fractionated (i.e. subjected to a separation step) and the determination may be performed on a fraction or subset of cells from or in the blood sample. As noted above such a fraction or subset may comprise leukocytes.

As well as blood and blood-derived samples, other haematopoietic samples may be used, that is any sample of haematopoietic tissue, e.g. bone marrow, but using a blood sample will generally be more convenient.

Further, as noted above, the blood sample (or blood-derived sample) may further be fractionated or subjected to a separation step to obtain a particular subset or fraction of nucleated blood cell or leukocyte. In other words a fraction or subset of (or containing) a particular blood cell type may be prepared and used in the determination step, or more particularly a particular type of leukocyte. Leukocytes may be classified into a number of different cell types, most notably granulocytes (polymorphonuclear leukocytes) which can be sub-divided into three types: neutrophils, basophils and eosinophils, and agranulocytes (mononuclear leukocytes) which include lymphocytes, monocytes and macrophages.

In certain particular embodiments the leukocyte may be a lymphocyte and especially a T-lymphocyte or T-cell. More particularly the T-cell may be a CD4+ T-cell (or helper T-cell). Thus a fraction or subset of cells may be prepared which contains (or comprises or consists essentially of) T-cells, and more particularly CD4+ T-cells. Thus the prediction method of the invention may comprise in step S1 determining a fraction of T-cells, or more specifically CD4+T-cells, from the blood sample that have lost chromosome Y.

As noted above, methods for separating different types of leukocytes are well known in the art. For example PBMCs and granulocytes may be separated by Ficoll-Paque centrifugation (Amersham Biosciences, Uppsala, Sweden) and T-cells e.g. CD4+ T-cells may be separated (e.g. from the PBMC fraction) by positive and/or negative selection based on cell-surface marker expression (e.g. CD4 expression). Kits and materials for performing such separations are commercially available (e.g. the CD4+ T cell Isolation Kit and CD4 microbeads from Miltenyi Biotech, Auburn, CA, USA). However, the separation is not limited to such methods and may be performed by other means e.g. by FACS sorting etc.

In a particular embodiment, the prediction of step S3 comprises predicting that the male subject has an increased risk of developing Alzheimer's disease if the fraction determined in step S1 is equal to or higher than the predefined threshold. Correspondingly, this step S3 preferably comprises predicting that the male subject does not have any increased risk of developing Alzheimer's disease if the fraction is lower than the predefined threshold.

The predefined threshold is thereby employed, in this embodiment, to differentiate those male subjects that do not have increased risk of developing Alzheimer's disease from those that have an increased risk of developing Alzheimer's disease as assessed based on the fraction of the cells that have lost chromosome Y.

The predefined threshold, to which the determined fraction of cells is compared in step S2, can be determined by the person skilled in the art based on determining the fraction of cells that have lost chromosome Y in multiple male subjects that currently are diagnosed to be healthy in terms of not having diagnosis of Alzheimer's disease. These male subjects can then be grouped into two main groups: 1) those that later on develop Alzheimer's disease and are thereby later on diagnosed as suffering from Alzheimer's disease and 2) those that never will develop Alzheimer's disease. The predefined threshold can then be set to discriminate between these two main groups.

In practical terms the threshold which is used may depend on the technique which is used to determine the loss of the Y chromosome. As described further below, the degree of LOY may be calculated from the median log R ratio values (measuring copy number) for genotyping using an array of probes in the male-specific region of chromosome Y (mLRR-Y). The mLRR-Y value may be used as a proxy for LOY, which is itself a proxy for the degree of mosaicism i.e. the percentage of cells that have lost the Y chromosome (see below). As described below, thresholds are set depending on mLRR-Y values. Specific mLRR-Y values are of course applicable only to the SNP-array technique and measurements and calculations as described below. However, as is clear from the discussion below, the thresholds may be set at the level of mosaicism (i.e. at the level of LOY) where the percentage of cells affected by LOY is not in the range of experimental variation and this is applicable to any means by which the LOY is measured or assessed (i.e. any technique or method for determining LOY). For example the threshold may be set at the level where the most robust statistical results are obtained (e.g. the most robust regression results, as described below). However, as also explained in more detail below, the threshold may be varied and can be set at a less stringent, or lower, level, for example the lowest value in the noise distribution (variation) may be used as the threshold. Thus, experimental variation, or experimental background noise, or more particularly the distribution thereof, may be used to set the predefined threshold for determination of LOY. The limit of experimental variation, and hence the threshold, may be set at different levels of stringency, as is clear from the detailed description below.

As shown in the detailed description of the study below (see in particular the legend to Figure 2 above), the lower 99% confidence interval (CI) of the distribution of expected experimental background noise (i.e. experimental variation) may be used as a pre-defined threshold to detect or determine LOY and this may be taken as representing an embodiment of a predefined threshold according to the invention. Accordingly, in an embodiment the method may be performed using the 99% confidence limits of a data set and the predefined threshold may be set according to (i.e. at or as) the lower limit of 99% confidence (i.e. using the lower 99% CI limit of the distribution of experimental variation (or alternatively expressed, of the distribution of experimental background noise) as the predefined threshold). Accordingly, in one preferred embodiment a predefined threshold may be the lower 99% CI of a LOY determination (i.e. a LOY frequency determination), more particularly of a LOY determination (or more particularly a LOY frequency determination) in a population of male subjects assessed for subsequent Alzheimer's disease development.

The data which is analysed to determine the fraction of cells with LOY and which may be used to set the predefined threshold may be corrected to allow or correct for any factors which may affect or confound the data, for example for variation between cohorts of data, and this may be performed according to principles well known in the art.

Thus, thresholds used in practice in a particular method of LOY determination may be translated into, or used to determine, a generally-applicable threshold which is not specific for or dependant upon a particular technique or method. For example as described further below, the percentage of cells which have lost chromosome Y may be calculated from the mLRR-Y values and a threshold may be set according to the percentage of cells in the sample, or more particularly the percentage of cells analysed, which are determined to have LOY (i.e. be affected by LOY).

A non-limiting but illustrative value of the predefined threshold is about 18 % (i.e. 18 % of cells). This value was determined as disclosed herein based on analysis of post-zygotic genetic aberrations in a large cohort from Uppsala Longitudinal Study of Adult Male (ULSAM).

It is, however, expected that the predefined threshold used in the comparison in step S2 could typically be selected in an interval from about 5 % to about 20 % (i.e. 5 to 20 % of cells). This means that if at least 5 % to 20 % of the cells from the biological sample have lost chromosome Y then the male subject is predicted to have an increased risk of developing Alzheimer's disease.

Other ranges for threshold values include 1-20, 1-15, 1-12, 1-10, 5-15, 5-12, 5-10, 7-15, 7-12, 7-10, 8-15, 8-12, 8-11, 9-12, and 9-11, or 1-8, 1-7 or 1-5 %, of cells from the biological sample that have lost chromosome Y, or more particularly of cells analysed. In one representative embodiment the threshold may be about 10% of cells. In another representative embodiment the threshold may be about 13% or about 13.1% of cells, as determined from the study below.

In a particular embodiment, the fraction of cells as determined in step S1 in Fig. 11 is preferably compared, in step S2, to a first predefined threshold and a second predefined threshold that is higher than the first predefined threshold. In such a case, the prediction as conducted in step S3 preferably comprises predicting that the male subject has an increased or moderate risk of developing Alzheimer's disease if the determined fraction is equal to or larger than the first predefined threshold but lower than the second predefined threshold. Step S3 preferably also comprises predicting that the male subject has a high risk of developing Alzheimer's disease if the fraction is equal to or larger than the second predefined threshold. Correspondingly, step S3 preferably also comprises predicting that the male subject does not have any increased risk of developing Alzheimer's disease if the fraction is lower than the first predefined threshold.

In this embodiment, the male subjects are differentiated into three groups: 1) those that do not have any increased risk of developing Alzheimer's disease, 2) those that have an increased but moderate risk of developing Alzheimer's disease, and 3) those that have a high or very high risk of developing Alzheimer's disease.

Thus, there is a relationship between the fraction of cells that have lost chromosome Y and the likelihood or risk of developing Alzheimer's disease. This means that the higher fraction of cells with LOY the higher the risk of developing Alzheimer's disease.

The first and second predefined thresholds can be determined as previously discussed herein. Experimental data from ULSAM indicate that suitable, but non-limiting, values of the first and second predefined thresholds could be 18 % and 35 %, respectively.

In an embodiment, the second predefined threshold is about twice the value of the first predefined threshold.

This concept can of course be extended to a case using more than two thresholds to differentiate male subjects into more than three different groups.

The determination of the fraction of cells that have lost chromosome Y can be performed according to various analysis methods and techniques. Generally, DNA and/or RNA from the cells in the biological sample is analyzed in order to determine presence or absence of chromosome Y in the cells. Thus DNA and/or RNA may be obtained from the cells to be analysed, e.g. from cells in the sample, or more particularly from a fraction or subset of cells in the sample. For instance, polymerase chain reaction (PCR) primers can be used to detect the presence or absence of any DNA sequence on chromosome Y and where the absence of the chromosome-Y-specific DNA sequence is regarded as LOY, which includes all variants of PCR methodology. Correspondingly, techniques can be used to detect presence or absence of mRNA transcribed from active genes present on chromosome Y. Absence of any significant detectable amounts of mRNA could then be regarded as LOY. Alternatively, the detection of LOY can be done on protein basis instead of or as an alternative to DNA and/or RNA analysis. In such a case, the presence or absence of a protein encoded by a gene on chromosome Y can be used to discriminate between those cells that have an intact chromosome Y and those cells that have lost chromosome Y.

Further non-limiting examples of analysis methods that can be used include single-nucleotide polymorphism (SNP) array analysis, RNA-assays, gene expression analysis, protein assays, epigenetic assays and various genetic methods, such as sequencing, comparative genomic hybridization (CGH) arrays, methylome assays and cytogenetic methods. It is also possible to visually detect LOY using microscopy methods.

Sequencing methods that can be used include basic and advanced sequencing methods, such as Maxam-Gillbert sequencing, Chain-termination methods, Shotgun sequencing and bridge PCT. Also, or alternatively, next-generation sequencing methods could be used to detect LOY and determine the fraction of cells that have lost chromosome Y. Examples of such next-generation sequencing methods include massively parallel signature sequencing (MPSS), polony sequencing, 454 pyrosequencing, Illumina sequencing, SOLID sequencing, Ion Torrent semiconductor sequencing, DNA nanoball sequencing, Heliscope single molecule sequencing, single molecule real time (SMRT) sequencing, nanopore DNA sequencing, tunneling currents DNS sequencing, sequencing by hybridization, sequencing with mass spectrometry, microfluidic Sanger sequencing, microscopy-based techniques, RNAP sequencing and *in vitro* virus high-throughput sequencing.

In a particular embodiment, the determination of the fraction in step S1 of Fig. 11 is based on SNP array analysis using data points derived from a pseudoautosomal region 1 (PAR1) region of chromosomes X and Y. In particular, the fraction may be determined based on a quantification of the data points derived from the PAR1 region by using a correlation between a Log R Ratio (LRR) in the PAR1 region of chromosome Y and an absolute deviation from an expected diploid B-allele frequency (BAF) value of 0.5 as further disclosed herein.

In another particular embodiment, the determination of the fraction in step S1 is based on sequencing of a DNA region present on chromosome Y.

Another aspect of the embodiments relates to the use of loss of chromosome Y (LOY) in cells from a biological sample obtained from a male subject as a genetic marker to predict whether the male subject has an increased risk of developing Alzheimer's disease.

Thus, in these embodiments LOY is used as a genetic marker for Alzheimer's disease prediction.

The above described method could be performed using a kit. Accordingly, a further aspect of the embodiments relates to a kit for predicting whether a male subject has an increased risk of developing Alzheimer's disease. The kit comprises means for determining a fraction of cells from a biological sample obtained from the male subject that have lost chromosome Y. The kit also comprises instructions for comparing the fraction obtained from the above mentioned means to a threshold defined or listed in the instructions. The kit further comprises instructions for predicting whether the male subject has an increased risk of developing Alzheimer's disease based on the comparison between the fraction and the predefined threshold.

The means for determining the fraction can include instruments, equipment and/or reagents needed to determine the fraction according to any of the previously mentioned methods or techniques. The instructions of the kit preferably specify the threshold, or the first and second predefined thresholds, and preferably also specify that the fraction should be compared to the threshold(s) and that the male subject is identified as having an increased risk of developing Alzheimer's disease if the determined fraction is equal to or exceeds the threshold. Correspondingly, the instructions could specify that the male subject does not have any increased risk of developing Alzheimer's disease if the determined fraction is lower than the threshold.

Various implementation embodiments and aspects will now be further described here below. The present embodiments are, in an aspect, directed to a method to predict the risk for Alzheimer's disease in men. In brief, we have discovered a strong association between somatic loss of chromosome Y (LOY) in, for instance, blood cells and risk for mortality in or running a risk of being diagnosed with Alzheimer's disease. This opens up for the prediction of individual Alzheimer's disease risks, herein denoted Alzheimer's Disease Risk Assessment from LOY-status (ADRAY). The ADRAY-evaluation, indicated by black box in Fig. 1, is based on assessment of LOY-status, achieved by estimation or determination of the fraction, percentage or degree of cells from a biological sample that have lost the chromosome Y. The LOY-status can be performed using genetic analysis, such as SNP-array, next-generation sequencing etc., or with other methodological approaches. When the LOY-status of a subject has been established, the risk for that subject to develop Alzheimer's disease can be assessed by comparing the degree of LOY to pre-defined risk-threshold(s) as described herein. The ADRAY-evaluation will, when used in large-scale screening of the general population of adult/senior/elderly men, be able to identify the approximate 10 % of late adult/elderly/old men that are at an increased risk for Alzheimer's disease as described below.

In a particular implementation example, a method of determining whether an individual has an increased risk for Alzheimer's disease comprises the steps of:
a) obtaining a biological sample from the individual;
b) determining the fraction, percentage or degree of cells from the biological sample of step a) that have lost the chromosome Y;
c) comparing the result obtained in step b) to pre-defined risk-threshold(s); and
d) determining whether the individual has an increased risk for Alzheimer's disease based on the comparison in step c).

In an embodiment, blood is the tissue type that is sampled and analyzed in the ADRAY-evaluation. In other embodiments, the tissue type that is sampled and analyzed may be skin, saliva, feces, urine, cerebrospinal fluid, hair or any other type of biopsy materials.

In an embodiment, senior/elderly men are sampled and analyzed in an analysis using the ADRAY-evaluation. In another embodiment, samples are collected at any other ages, such as young or middle-aged subjects/cohorts. Thus in certain embodiments the male subject may be at least 40, 45, 50, 55, 60, 65 or 70 years of age, that is representing more advanced or senior age, In other embodiments the male subject may at least 20, 25, 30, or 35 years of age, representing also subjects of younger age. Age ranges of subjects may be between any of these integers.

As is clear from the more detailed description below, it may be advantageous to test male subjects in a longitudinal way, that is to perform repeated tests over time, as the subject ages.

In an embodiment, DNA is analyzed in an analysis using the ADRAY-evaluation. In other embodiments, any other sources of biological material or types of molecules are examined to estimate the degree of LOY, such as RNA-assays, gene-expression, protein-assays, and epigenetic-assays etc.

In an embodiment, Illumina SNP-array technology is performed to produce the data that is analyzed in an analysis using the CRAY-evaluation. In other embodiments, any other genetic (or other) methods, such as next-generation sequencing, CGH-arrays, RNA-assays, gene-expression technology, methylome technology, protein-assays, cytogenetic and microscopy methods etc., which can be used to produce the data that, are used to estimate the degree of LOY.

In an embodiment, the assessments of chromosome Y status, i.e. LOY-assessments, are performed using the MAD-software [14, 15] on the SNP-array-data prior to the ADRAY-evaluation in an analysis. In other embodiments, also or alternatively any other software or methods to perform the LOY-assessment, such as any methods to determine or estimate the levels of somatic mosaicism, including manual calling, are used.

In an embodiment, the risk of Alzheimer's disease is predicted in an analysis using the ADRAY-evaluation.

The herein described ADRAY-evaluation has numerous potential clinical applications including, but not limited to, prediction of individual Alzheimer's disease risk from LOY-status. Such predictions could be made in large screening programs in the population or in smaller scale during medical check-ups or in personal examination programs. Regardless of the clinical or other settings, the method described will be able to identify subjects with an increased risk to develop Alzheimer's disease (-10 % in the general population of elderly men according to our present estimates) based on the LOY-status.

A positive result from a ADRAY-evaluation could result in a remittance for full clinical (neurological) evaluation. If these -10 % with detectable LOY would be remitted for neurological evaluation, patients with Alzheimer's disease in early and/or late progression could be found. Such detection, and in particular early detection, would generally entail more efficient treatment strategies against the Alzheimer's disease. In this context, it is worth mentioning that 50 % median survival in men affected with LOY, i.e. loss of chromosome Y in ≥ 35 % of blood cells, was only 4 years, which should be compared to 11 years average survival in LOY-free men (Fig. 5A). Hence, 7 years difference in mean survival.

Subjects with somatic LOY and, thus, an increased risk for Alzheimer's disease, but with no detectable Alzheimer's disease, could be recommended for further ADRAY-evaluation at later age, see Fig. 1. Measurement of LOY in blood could therefore become a useful predictive biomarker of Alzheimer's disease.

Hence, a clinical use of the present embodiments is to identify individuals with an increased risk of developing Alzheimer's disease and to target these individuals for more frequent medical examinations.

Further, another clinical use of the present embodiments is to identify suitable treatment regimens for patients at an early state of Alzheimer's disease progression.

The above described applications of ADRAY, as well as any other applications of ADRAY, could be performed using a kit. Such a kit could be composed of some or all of the following components:
- any equipment suitable to detect loss of chromosome Y (LOY) from biological tissue(s);
- different approaches or algorithms suitable to analyze the data obtained using the above mentioned equipment and infer the LOY-status from the data;
- instructions to relate and compare the inferred LOY-status to pre-defined threshold(s) of risks for Alzheimer's disease; and
- instructions and manuals for the use of the kit.

Following on from this showing that determining the loss of chromosome Y may be an important predictive tool for predicting risk of Alzheimer's disease, or indeed a risk of earlier mortality, it becomes clear that that LOY may be important in the development of Alzheimer's disease, and that accordingly it may be useful to study the fraction of cells which have lost chromosome Y to help elucidate the underlying mechanism which leads to the observed increased risk of developing Alzheimer's disease, and the development of Alzheimer's disease itself. This represents a further aspect of the invention, namely the proposal that LOY may be studied or assessed in cells (namely cells that would normally be expected to have a Y chromosome) with respect to studying the effect of the LOY on Alzheimer's disease risk and the development of Alzheimer's disease. Thus, in a further aspect of the invention it is proposed that loss of chromosome Y be determined in cells (which may contain a Y chromosome) with a view to determining how LOY increases the risk of developing Alzheimer's disease or how LOY may lead to Alzheimer's disease. In such cases the LOY determination may be performed not just on cells or samples from a male subject undergoing an Alzheimer's disease risk assessment, but on any cell that may contain a Y chromosome, including cells from a male subject who has previously undergone a risk prediction assessment according to the present invention, who may or may not have developed Alzheimer's disease. Thus the studied cell may be a diseased cell from such a subject or a non-diseased cell. It may be a cell from an animal model e.g. an Alzheimer's disease model. It may further be a cell derived from a cell obtained from a subject who has undergone the risk prediction assessment, for example cells from the subject may be cultured and/or treated e.g. transformed for use in future study. Thus the cell may be from a cell-line, including from a cell line generated from a subject who has undergone an Alzheimer's disease risk prediction assessment according to the methods of the invention above.

In one sense, it can be seen that the invention provides use of LOY in a cell to investigate the association between risk of developing Alzheimer's disease and the Y chromosome or loss thereof, or the association between Alzheimer's disease and the Y chromosome or loss thereof.

In one embodiment of this aspect, the invention provides a method of investigating the association between Alzheimer's disease, or the risk of developing Alzheimer's disease (e.g. the onset of Alzheimer's disease), said method comprising determining the loss of chromosome Y in a cell (particularly determining the fraction of cells in a sample which have lost chromosome Y). The sample may be any sample of cells that may contain a chromosome Y (and particularly which may be subject to LOY), but in a particular embodiment may be cells obtained from a subject who has undergone an Alzheimer's disease risk prediction according to the method of the invention as defined and described hereinbefore, or cells derived from such obtained cells.

### Brief description of the study

When studying the impact of post-zygotic genetic aberrations in a large cohort from Uppsala Longitudinal Study of Adult Men (ULSAM) a correlation between somatic loss of chromosome Y (LOY) and risk for Alzheimer's disease was discovered. The ULSAM study was initiated in 1970 [16], where 2322 men, born in Uppsala in 1920-1924, participated at the age of 50. The study is investigating a wide range of clinical phenotypes, including cardiovascular diseases, cognitive deficits and dementia as well as cancer history from the National Cancer Registry and the Swedish Civil Registry. Major re-examinations and sampling have been made at ages 60, 70, 77, 82 and 88 years.

According to the embodiments, peripheral blood DNA from 1153 ULSAM participants was genotyped using high-resolution 2.5MHumanOmni SNP-beadchip from Illumina. The population-based ULSAM cohort has extensive phenotypic information of naturally aging men that were clinically followed for >40 years. We studied DNA sampled at an age window of 70.7-83.6 years. Scoring of structural genetic variants was focused on post-zygotic, acquired changes, such as deletions, copy number neutral loss of heterozygozity (CNNLOH), also called acquired uniparental disomy (aUPD), and gains, as described previously [1, 17]. In these analyses 551 autosomal structural variants were uncovered, including 70 deletions, 16 CNNLOH and 465 gains.

Strikingly, the most frequent somatic variant was the loss of chromosome Y (LOY), see Fig. 2. In brief, the degree of LOY was calculated for each subject and suggested considerable inter-individual differences regarding the proportion of cells with nullisomy Y. A conservative estimate of the frequency of LOY in the cohort was at least 8.2 % (see below). At this threshold, >18 % of cells in affected participants would be expected to have nullisomy Y. The effects from the above described structural variants on all-cause mortality, cancer mortality and Alzheimer's disease mortality were examined by Cox proportional hazards regression. In survival analyses, 1135 participants free from Alzheimer's disease prior to sampling as well as 968 participants free from Alzheimer's disease and cancer prior to sampling were studied. These tests revealed that LOY was by far the most important risk factor for Alzheimer's disease mortality, and showed that median survival (50 % probability of survival) in the group of men with LOY was about 7 years shorter, compared to controls. Thus, the human chromosome Y is important for biological processes beyond sex determination and reproduction. It has been known for half a century that elderly males frequently lose chromosome Y in normal hematopoietic cells [3, 4]. The clinical consequences of this aneuploidy have been unclear and the prevailing consensus suggests that this mutation should be considered phenotypically neutral and related to normal aging [5-10]. Our results suggest otherwise and clearly point out that LOY as well as other acquired chromosomal abnormalities are associated with negative phenotypic consequences for the affected men.

### Frequency of men with LOY in the population

We found that somatic loss of chromosome Y (LOY) was the most common structural genetic aberration in the studied ULSAM-cohort of 1153 elderly men, see Fig. 2. An ultra-conservative estimate of the LOY-frequency in this cohort yields that 8.2 % are affected as shown in Fig. 2. However, using a 99 % confidence interval for estimating the LOY-frequency in the cohort, we see that 14.7 % are affected, see Fig. 2. Both of these two mentioned frequencies are very high compared to other known somatic mutations in the general population.

### Frequency of cells with LOY within subjects

The frequency, or fraction, percentage, degree, of cells with loss of chromosome Y (LOY) was estimated in every subject prior to further analyses and should not be confused with the frequency of men with LOY in the studied population described above. An estimation of the percentage of blood cells affected with LOY was performed through analysis of SNP-array data from the pseudoautosomal region 1 (PAR1) of chromosomes X/Y using MAD-software [14], see Fig. 3. PAR1 is the largest of the PARs, i.e. regions with homologous sequences on chromosomes X and Y, with coordinates 10001-2649520 on Y and 60001-2699520 on X. MAD-software is a tool for detection and quantification of somatic structural variants from SNP-array data, which uses diploid B-allele frequency (BAF) for identification and Log R Ratio (LRR) for quantification of somatic variants and is not originally intended for analyses of chromosome Y data. By using the correlation between the LRR in the PAR1-region of Y and the delta-BAF, i.e. the absolute deviation from the expected BAF-value of 0.5 in heterozygous probes, of the PAR1-region of X/Y, see Fig. 3A, the MAD-quantification of the diploid PAR1 region on chromosomes X/Y could be used to calculate the percentage of cells affected with LOY, see Fig. 3B. Furthermore, the level of LOY, i.e. percentage of affected cells, is not a static, binary feature but is rather changing dynamically over time, see Fig. 4. Through analysis of unique longitudinal samples we described a pattern, in which the number of affected cells is increasing with time within subjects. These observations suggest that the cells affected with LOY are having a proliferative advantage compared to wildtype cells.

### Correlations between LOY, mortality and Alzheimer's disease

Age-related loss of chromosome Y (LOY) has been known to occur for decades in normal hematopoietic cells [3, 4] but the phenotypic consequences of LOY have been elusive [5-10]. We have shown herein that somatic loss of chromosome Y (LOY) in the blood cells from men were correlated with reduced survival. Median 50 % survival among men in the group affected with LOY was ∼4 years whereas men in the group without detectable LOY had a 50 % median survival of ∼11 years, see Fig. 5. Hence, the 50 % median survival was 7 years shorter in LOY-men.

Specifically, the primary survival analysis using Cox proportional hazards regression and a continuous estimator of LOY (i.e. the mLRR-Y) performed in 1153 ULSAM subjects showed that the risk of all-cause mortality was associated with LOY (HR=2.15, 95 % CI=1.20-3.84, p=0.0099, events=764). After scoring (zero or one) the men based on their degree of LOY (i.e. mLRR-Y < -0.4, Fig. 2), the survival analyses further illustrated that men with LOY are at a higher risks for all-cause mortality (HR=1.92, 95 % CI=1.25-2.96, p=0.0031, events=764, Fig. 5A). To evaluate the risk for mortality in AD as an effect of LOY, a refined survival analysis using 1135 subjects not diagnosed with AD prior to genotyping was performed. Using the same scoring based on the level of LOY as above (i.e. mLRR-Y < -0.4, Fig. 2), showed that the risk for mortality in AD is strongly correlated with LOY (HR=3.27, 95 % CI=1.29-8.30, p=0.0128, events=124, Fig. 5B). Further exploratory survival analyses using other thresholds for mLRR-Y further strengthen this conclusion (Fig. 6). Hence, the ADRAY-evaluation could predict the risk for Alzheimer's disease using DNA sampled from blood cells. As illustrated in Fig. 6, a higher degree of LOY within subjects also seemed to entail larger risks. Measurement of LOY in blood can therefore be a useful predictive biomarker of Alzheimer's disease development. Risks of morbidity and mortality from Alzheimer's disease are shown in Fig 5 and 6.

### Assessment of LOY-status

After the genetic analyses, or alternative detection method, the loss of chromosome Y-status is assessed from the data produced before the ADRAY-evaluation, see Fig. 1. The LOY-status is a proxy for degree of mosaicism, i.e. the percentage of cells, in the analyzed tissue that has lost the Y chromosome. Such LOY-status can be estimated from median Log R Ratio on chromosome Y (mLRR-Y). The Log R Ratio is the type of data from the Illumina SNP-array analysis that shows the copy-number status. The mLRR-Y for each subject is calculated as the median LRR in the male specific region of chromosome Y, between PAR1 and PAR2. Estimations of the percentages of cells that are affected by loss of chromosome Y (LOY) may be obtained by SNP-array and the estimation of percentage can be performed using MAD-software [15]. The exact method for estimating the percentage of cells carrying the aberrations is, however, not of importance for the present embodiments. Any other software or algorithms could also suffice to produce an estimation of degree of mosaicism that will later be used to perform the assessment of LOY-status and risk for Alzheimer's disease.

### Thresholds in the degree of LOY for the ADRAY-evaluation

For the ADRAY-evaluation two thresholds in mLRR-Y, T1 and T2 were defined. The first threshold (T1, line at mLRR-Y=-0.139 in Fig. 2) was set at the level of mosaicism where the percentage of cells affected by loss of chromosome Y was clearly not within the expected experimental variation. At this level 18 % or more of the examined cells within a sample was affected with LOY. The second threshold (T2, line at mLRR-Y=-0.4 in Fig. 2) was set at the lowest level of mosaicism where Cox survival analyses were giving robust regression results, see Fig. 6. Setting the T2 threshold at a higher level of mosaicism (such as mLRR-Y=-0.5 or mLRR-Y=-0.6) gives even higher hazard ratios in the analyses (Fig. 6). However, since such strict settings will not include subjects with moderate levels of LOY between -0.4 and -0.5 (or -0.6), the significant threshold of T2 at -0.4 was preferred. In analyses performed in datasets with T2 set at a lower level of mosaicism, such as mLRR-Y=-0.3 or mLRR-Y=-0.2, the low level of mosaicism was giving a smaller biological effect and, thus, lower hazard ratios, see Fig. 6. At mLRR-Y=-0.4, 35 % or more of the examined cells within a sample was affected with LOY. It is appreciated that the thresholds defined above are based on the data presently available and a person skilled in the art would understand the potential need to adjust the thresholds depending on the results of analyses performed in larger cohorts.

### Prediction of risk with ADRAY

ADRAY-evaluation of Alzheimer's disease risk was performed by comparing individual degree of somatic loss of chromosome Y to pre-defined risk threshold values, T1 and T2. Subjects with detected somatic LOY where characterized as having increased risk of Alzheimer's disease if mLRR-Y<T1. Our results indicated that subjects in this group were at an increased risk to develop Alzheimer's disease as compared to subjects with mLRR-Y>T1. Subjects with even higher degree of somatic LOY were characterized as having great risk of Alzheimer's disease if mLRR-Y<T2. Our results showed that subjects in this group are at a significantly higher risk to develop non-hematological Alzheimer's disease as compared to subjects in which mLRR-Y>T2, see Fig. 5, HR=3.55, CI=1.52-8.30, p=0.003.

In an embodiment, as illustrated in Fig. 1, subjects with mLRR-Y<T1 could be remitted for neurological evaluation. The thresholds were defined from the empirical data of loss of chromosome Y (LOY) and risk for different types of Alzheimer's disease, but they are not static. Analyses indicated that optimal values for the T1 and T2 thresholds could be at -0.139 and -0.4, respectively, as measured in mLRR-Y. Analyses of larger cohorts and using other techniques or methods for LOY-status assessment, may lead to a gradual fine-tuning of optimal values for the T1 and T2 thresholds. An embodiment is directed to the prediction of Alzheimer's disease risk based on individual degree of loss of chromosome Y (LOY) in relation to pre-defined threshold(s), irrespective of the exact value of the threshold(s).

### Tissue analyzed

The correlation between somatic loss of chromosome Y (LOY) and the risk for Alzheimer's disease were based on analyses performed using DNA extracted from samples of whole peripheral blood in elderly men. However, any other tissue source of DNA, taken at any age, could be applicable for the ADRAY-evaluation.

### Methods for detection of LOY

In an embodiment, genetic experiments performed using SNP-array technology are used. However, the present embodiments are not limited to any particular detection method. Any suitable method or technology could also suffice to produce the data useful to perform the Alzheimer's disease-risk-assessment from LOY-status. In certain subjects it may become useful to repeat the initial genetic experiments and/or to perform secondary or validation experiments before the ADRAY-evaluation.

### EXAMPLES

Four examples are presented in which the use and potential benefits of ADRAY-evaluations are illustrated. In these examples, an ADRAY-evaluation could have had an impact on earlier diagnosis of Alzheimer's disease predicted in patients with somatic loss of chromosome Y (LOY). Examples 1-3 are based on the real data with medical history and genotyping data from three longitudinally monitored ULSAM participants, who were diagnosed with AD. Their treatment would have been aided by an early LOY-detection and ADRAY-evaluation. In the last example, the benefits of ADRAY-evaluation are illustrated with a hypothetical AD patient using two different scenarios. The first of these two scenarios is similar to examples 1-3, since the patient has a detectable risk from LOY-status but still later dies from disease. In the second scenario, the AD risk is detected through LOY-status assessment and ADRAY-evaluation. The subsequent clinical evaluation diagnosed the patient and the disease could have been treated in a very early phase. Further, in this fictional example, the frequency of defective LOY-cells could be reduced using various future treatment alternatives, such as stem cell therapeutics.

### Example 1

This example shows a patient named ULSAM-364, who was genotyped on SNP-array at two different ages, 77 and 89 years old, and he subsequently died from Alzheimer's disease (AD) at the age of 89. Somatic loss of chromosome Y (LOY) was present at both genotyping ages with 25 % and 53 % of affected cells, respectively. An additional ADRAY-evaluation in between the ages of 77 and 89 would have predicted a risk of AD. See Fig. 7.

### Example 2

This example 2 shows a subject ULSAM-41 who was genotyped using SNP-array at two ages, 83 and 88 years old, and died at the age of 90 having a diagnosis of Alzheimer's disease. Somatic loss of chromosome Y (LOY) was present at both genotyping ages with 20 % and 45 % of affected cells, respectively. An ADRAY-evaluation at the age of 85 or later would have predicted an increased risk of AD. See Fig. 8.

### Example 3

This example displays a subject ULSAM-569 who was genotyped on SNP-arrays at three ages, 75, 82 and 88 years, and died at the age of 89 with a diagnosis of Alzheimer's disease. Somatic loss of chromosome Y (LOY) was present at all genotyping ages with 26 %, 43 % and 72 % of affected cells, respectively. An ADRAY-evaluation at the age of 75 years or later would have predicted an increased risk of AD. Screening of blood sampled at even younger ages would probably entail an even earlier risk of future diagnosis of AD. See Fig. 9.

### Example 4

This hypothetical example illustrates the benefits of ADRAY-evaluation using a hypothetical Alzheimer's patient in two different scenarios, see Fig. 10. In the first scenario, no ADRAY-evaluation is performed and the subject dies from AD at the age of 75. This scenario is very similar to examples 1-3 and the clinical reality of contemporary AD clinical management. In the alternative scenario, the ADRAY-evaluation performed at the age of 65 detects the disease risk, i.e. LOY. In this patient a clinical evaluation could diagnose AD that was treated in a very early phase. Furthermore, we anticipate that the frequency of defective LOY-cells can be reduced using a variety of future treatment strategies, such as stem cell therapy or adjuvant stimulation. See Fig. 10.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

### Example 5

This example shows that the lower 99% CI may be used to set a threshold for predicting Alzheimer's disease risk. Analysis of LOY was carried out in three independent prospective cohorts; ULSAM (described above), the PIVUS (Prospective Investigation of the Vasculature in Uppsala Seniors) (http://www.medsci.uu.se/pivus)[23] and the TwinGene cohort (a subset of twin pairs from the Swedish Twin Registry, where both twins participated in Screening Across the Lifespan Twin (SALT) (21-23).

Uppsala Longitudinal Study of Adult Men (ULSAM) was started in 1970-1974, when all fifty year old men living in Uppsala County, Sweden, were invited to a health survey, initially focusing at identifying risk factors for cardiovascular disease (http://www2.pubcare.uu.se/ULSAM/index.htm). Out of a total of 2841 men born in 1920-1924, 2322 (82%) agreed to participate in the study. Since then, the cohort has been reinvestigated several times at 60, 70 (when the first blood samples for DNA extraction were collected), 77, 82, 88, 91 and 93 years. For the present analysis, peripheral blood DNA from 1153 men, who provided the blood sample at the 70-77 years investigation and were successfully genotyped, was used, as recently described (21). The PIVUS study (Prospective Investigation of the Vasculature in Uppsala Seniors) (http://www.medsci.uu.se/pivus) was begun in 2001 with the primary aim of investigating the predictive power of various measurements of endothelial function and arterial compliance. Eligible participants were all aged 70 and were living in the community of Uppsala, Sweden. The subjects were randomly chosen from the community register, and 1,016 men and women participated. Two reinvestigations of the PIVUS cohort were undertaken, starting in the spring of 2006 and in the spring of 2011 at the ages of 75 and 80 years, respectively. Here we have used 488 male DNA samples collected at the age of 70 years, which were successfully genotyped and described recently (21). The TwinGene cohort is a subset of twin pairs from the Swedish Twin Registry, where both twins participated in Screening Across the Lifespan Twin (SALT) study, were willing to provide a blood sample as well as responded to a questionnaire, conducted between 2005 and 2008. In the current study we used the male member of opposite sex dizygotic twin pairs and randomly selected one member of male twin pairs to be included. In total 4373 male samples were successfully genotyped and included. Sorting of subpopulations of cells from peripheral blood (granulocytes, CD4+ T-lymphocytes and CD19+ B-lymphocytes) as well as culturing of skin fibroblasts from ULSAM subjects 340, 973 and 1412 was performed as described previously (18).

### LOY estimation and data quality control

SNP genotyping with Illumina beadchips was performed according to the instructions from the manufacturer. All SNP genotyping and next-generation sequencing was performed by the SNP&SEQ Technology Platform in Uppsala, Sweden, as described previously (18, 21). The results from SNP genotyping consist of (in addition to SNP calls) two main data tracks for each SNP probe: Log R Ratio (LRR) and B-allele frequency (BAF). The degree of LOY in each participant was estimated by calculating the median LRR value (mLRR-Y) in the male-specific region of chromosome Y (MSY): a -56 Mb region between pseudoautosomal regions 1 and 2 (PAR1 and PAR2) on chromosome Y(chr. Y: 2,694,521-59,034,049, hg19/GRCh37) (21). The 2.5M- and HumanOmniExpresschips used here contain approximately 2.560 and 1.690 SNP probes in the MSY region, respectively.

All included experiments passed strict quality control (18, 21). In addition, we calculated the sdLRR1 (i.e. the standard deviation of Log R Ratio of probes on chromosome 1) for all experiments, as recommended by Illumina. The sdLRR1 was added as a covariate ("Genotyping quality") in regression analysis.

### Statistical analyses and correction of mLRR-Y values

All statistical analyses were performed with the R software (v 3.0.2) (22). The mLRR-Y values of all participants were corrected prior to statistical analyses using a cohort-specific correction constant to improve the comparability between the three cohorts. As correction constants, the cohort specific median of the mLRR-Y values was used.

### Estimation of LOY frequency in TwinGene, ULSAM and PIVUS

To find an appropriate threshold for estimation of the LOY frequencies, the contribution from experimental noise in the mLRR-Y values was estimated as previously described (21). The total variation in mLRR-Y values in the three cohorts (bars to the left of the lower 99% CI dashed line in Figs. 13-15) consisted of a signal from LOY and a signal from experimental variation. We assumed that the experimental noise in mLRR-Y was distributed in a nonskewed fashion and that variation in the positive tail of the mLRR-Y distribution was all experimentally induced. This latter assumption was realistic since no validation experiments could confirm any suspected cases of gain of chromosome Y (21). The experimental distribution (white bars) was generated by imposing the observed variation in the positive tail into a reflected negative tail in two steps. First, the peak in the histogram of mLRR-Y (vertical coloured lines in Figs. 13-15) was determined with a kernel density estimation method (kernel medial) using the Density function in R. The bandwidth "SJ" was used and the bin with the highest value (i.e., distribution peak) in the smoothed distribution was selected as the local median (21). Next, the observations in the positive tail were mirrored over the kernel-derived median to create the negative tail. The lower 99% confidence interval (CI) of these distributions of experimental noise (white bars) was used as threshold for LOY-scoring. Hence, the LOY frequency within each cohort was calculated as the fraction of men with mLRR-Y values lower than the 99% CI of the distribution of experimental noise (coloured bars). The percentage of cells affected with LOY at the lower 99% confidence limit (Figs. 13-15) was estimated from the five ULSAM participants (subjects 630, 261, 973, 76 and 1071) with mLRR-Y values closest and under this threshold. We used the B allele frequencies from SNP probes located in the PAR1 region for this estimation, as previously described (21). Estimation of LOY-frequency in the ULSAM, PIVUS and TwinGene cohorts from SNP-array data after accounting for the experimentally induced variation (white bars), as previously described (21), and detailed above are shown in Figures 13-15. The bars in the left tail of the mLRR-Y distribution represent the men that had mLRR-Y values below the lower 99% confidence interval of the experimental noise distribution and scored with LOY. In the three different cohorts the mLRR-Y is similar and represents about 10% of cells affected with LOY.

### Example 6

This example illustrates how LOY varies with age and in different cellular compartments of blood in ULSAM subjects 340, 973 and 1412 (Fig 16-18 respectively). LOY is a progressive phenomenon, clearly detectable at age 90 (subject 340), 86 (subject 973) and 72 (subject 1412) in whole blood. Interestingly, among the blood compartments, the granulocyte component is the most affected by LOY. These ULSAM participants are among about 250 survivors still alive at the age of 91 years, from the original ULSAM cohort of about 2300 men at the start of ULSAM in 1970, when all participants were at 50 years of age. The subjects had no cancer or AD diagnosis at time of sampling. The CD4+ cells were not affected with LOY in these subjects who survived at least until the age of 91 years of age and with no cancer or AD diagnosis at this age. This supports the notion that CD4+ cells in whole blood are important to fight back cancer and AD development in all tissues and organs of the body, potentially via the function of immunosurveillance.

### REFERENCES

1. Forsberg, L.A., D. Absher, and J.P. Dumanski, Non-heritable genetics of human disease: spotlight on post-zygotic genetic variation acquired during lifetime. J Med Genet, 2013. 50(1): p. 1-10.
2. Lambert, J.C., et al., Meta-analysis of 74,046 individuals identifies 11 new susceptibility loci for Alzheimer's disease. Nat Genet, 2013. 45(12): p. 1452-8.
3. Jacobs, P.A., et al., Change of human chromosome count distribution with age: evidence for a sex differences. Nature, 1963. 197: p. 1080-1.
4. Pierre, R.V. and H.C. Hoagland, Age-associated aneuploidy: loss of Y chromosome from human bone marrow cells with aging. Cancer, 1972. 30(4): p. 889-94.
5. Nowinski, G.P., et al., The frequency of aneuploidy in cultured lymphocytes is correlated with age and gender but not with reproductive history. Am J Hum Genet, 1990. 46(6): p. 1101-11.
6. Loss of the Y chromosome from normal and neoplastic bone marrows. United Kingdom Cancer Cytogenetics Group (UKCCG). Genes, chromosomes cancer, 1992. 5(1): p. 83-8.
7. Wiktor, A., et al., Clinical significance of Y chromosome loss in hematologic disease. Genes, chromosomes & cancer, 2000. 27(1): p. 11-6.
8. Wong, A.K., et al., Loss of the Y chromosome: an age-related or clonal phenomenon in acute myelogenous leukemia/myelodysplastic syndrome? Archives of pathology & laboratory medicine, 2008. 132(8): p. 1329-32.
9. Wiktor, A.E., et al., The significance of isolated Y chromosome loss in bone marrow metaphase cells from males over age 50 years. Leukemia research, 2011. 35(10): p. 1297-300.
10. Jacobs, P.A., et al., Male breast cancer, age and sex chromosome aneuploidy. Br J Cancer, 2013. 108(4): p. 959-63.
11. Waldemar, G., et al., Recommendations for the diagnosis and management of Alzheimer's disease and other disorders associated with dementia: EFNS guideline. Eur J Neurol, 2007. 14(1): p. e1-26.
12. Bonin-Guillaume, S., et al., [The economical impact of dementia]. Presse Med, 2005. 34(1): p. 35-41.
13. Meek, P.D., K. McKeithan, and G.T. Schumock, Economic considerations in Alzheimer's disease. Pharmacotherapy, 1998. 18(2 Pt 2): p. 68-73; discussion 79-82.
14. Gonzalez, J.R., et al., A fast and accurate method to detect allelic genomic imbalances underlying mosaic rearrangements using SNP array data. BMC bioinformatics, 2011. 12(1): p. 166.
15. Pique-Regi, R., A. Caceres, and J.R. Gonzalez, R-Gada: a fast and flexible pipeline for copy number analysis in association studies. BMC Bioinformatics, 2010. 11: p. 380.
16. Hedstrand, H., A study of middle-aged men with particular reference to risk factors for cardiovascular disease. Ups J Med Sci Suppl, 1975. 19: p. 1-61.
17. Forsberg, L., et al., Age-related somatic structural changes in the nuclear genome of human blood cells. Am J Hum Genet, 2012. 90(2): p. 217-28.
18. Forsberg, L. et al, Mosaic loss of chromosome Y in peripheral blood is associated with shorter survival and higher risk of cancer. Nature Genetics 46, 624-628 (2014).
19. R Development Core Team, R: A language and environment for statistical computing. (R Foundation for Statistical Computing, Vienna, Austria, 2012).
20. L. Lind, N. Fors, J. Hall, K. Marttala, A. Stenborg, Arterioscler Thromb Vasc Biol 25, 2368 (Nov, 2005).
21. P. Lichtenstein et al., Twin Res Hum Genet 9, 875 (Dec, 2006).
22. P. K. Magnusson et al., Twin Res Hum Genet 16, 317 (Feb, 2013).
23. P. Lichtenstein et al., J Intern Med 252, 184 (Sep, 2002).
24. F. Faggioli et al., Human Molecular Genetics 21, 24 (Sep, 2012).

## Claims

1. A method of predicting whether a male subject has an increased risk of developing Alzheimer's disease, comprising:
determining (S1) a fraction of cells from a biological sample obtained from said male subject that have lost chromosome Y;
comparing (S2) said fraction to a predefined threshold; and
predicting (S3) whether said male subject has an increased risk of developing Alzheimer's disease based on said comparison between said fraction and said predefined threshold, wherein;
said male subject has an increased risk of developing Alzheimer's disease if said fraction is equal to or higher than said predefined threshold; or
said male subject does not have any increased risk of developing Alzheimer's disease if said fraction is lower than said predefined threshold.

2. The method according to claim 1, wherein determining (S1) said fraction comprises determining (S1) a fraction of somatic cells from said biological sample that have lost chromosome Y.

3. The method according to claim 1 or claim 2, wherein the biological sample is a blood, blood-derived, lymph, saliva, urine, faeces, CSF, skin or hair sample.

4. The method according to any one of claims 1 to 3, wherein the cells in step S1 are nucleated blood cells from a blood sample obtained from said male subject, wherein determining (S1) said fraction comprises determining (S1) a fraction of nucleated blood cells from said blood sample that have lost chromosome Y.

5. The method of any one of claims 1 to 4, further comprising obtaining a subset or fraction of cells from the biological sample and determining (S1) a fraction of cells from said subset or fraction that have lost chromosome Y.

6. The method according to any one of claims 1 to 5, wherein determining (S1) said fraction comprises determining a fraction of CD4+T-cells that have lost chromosome Y.

7. The method according to any one of claims 1 to 6, wherein said predefined threshold lies in the range 5 to 20% of cells.

8. The method according to any one of claims 1 to 7, wherein comparing (S2) said fraction comprises comparing (S2) said fraction to a predefined threshold of about 18 %.

9. The method according to any one of the claims 1 to 7, wherein comparing (S2) said fraction comprises comparing (S2) said fraction to a predefined threshold of about 10 %.

10. The method according to any one of claims 1 to 9, wherein a predefined threshold is set at the lower 99% confidence interval of the distribution of expected experimental background noise in a determination of frequency of loss of chromosome Y.

11. The method according to any of the claims 1 to 10, wherein
comparing (S2) said fraction comprises comparing (S2) said fraction to a first predefined threshold and a second predefined threshold that is higher than said first predefined threshold, and
predicting (S3) whether said male subject has an increased risk of developing Alzheimer's disease comprises:
predicting that said male subject does not have any increased risk of developing Alzheimer's disease if said fraction is lower than said first predefined threshold;
predicting that said male subject has an increased risk of developing Alzheimer's disease if said fraction is equal to or larger than said first predefined threshold but lower than said second predefined threshold; and
predicting that said male subject has a high risk of developing Alzheimer's disease if said fraction is equal to or larger than said second predefined threshold.

12. The method according to claim 11, wherein comparing (S2) said fraction comprises comparing (S2) said fraction to said first predefined threshold of about 18 % and said second predefined threshold of about 35 %.

13. The method according to any of the claims 1 to 12, wherein determining (S1) said fraction comprises determining (S1) said fraction based on;
(i) a single-nucleotide polymorphism, SNP, array analysis using data points derived from a pseudoautosomal region 1, PAR1, region of chromosomes X and Y; or
(ii) quantification of said data points derived from said PAR1 region on chromosomes X and Y by using a correlation between a Log R Ratio, LRR, in said PAR1 region of chromosome Y and an absolute deviation from an expected diploid B-allele frequency, BAF, value of 0.5.

14. The method according to any of the claims 1 to 12, wherein determining (S1) said fraction comprises determining (S1) said fraction based on sequencing of a DNA region present on chromosome Y.

15. The method according to any of the claims 1 to 14 wherein the subject is healthy, not suffering from Alzheimer's disease or not identified as having Alzheimer's disease.

16. Use of loss of chromosome Y in cells from a biological sample obtained from a male subject as a genetic marker to predict whether said male subject has an increased risk of developing Alzheimer's disease.

## Patentansprüche

1. Verfahren zur Vorhersage, ob eine männliche Person ein erhöhtes Risiko für die Entwicklung der Alzheimerkrankheit aufweist, umfassend:
Bestimmen (S1) eines Anteils von Zellen aus einer biologischen Probe, die von der männlichen Person erhalten wurde, die das Y-Chromosom verloren haben;
Vergleichen (S2) des Anteils mit einem vordefinierten Schwellenwert; und
Vorhersagen (S3), ob die männliche Person ein erhöhtes Risiko für die Entwicklung der Alzheimerkrankheit aufweist, basierend auf dem Vergleich zwischen dem Anteil und dem vordefinierten Schwellenwert, wobei;
die männliche Person ein erhöhtes Risiko für die Entwicklung der Alzheimerkrankheit aufweist, wenn der Anteil gleich oder höher als der vordefinierte Schwellenwert ist; oder
die männliche Person kein erhöhtes Risiko für die Entwicklung der Alzheimerkrankheit aufweist, wenn der Anteil niedriger als der vordefinierte Schwellenwert ist.

2. Verfahren nach Anspruch 1, wobei Bestimmen (S1) des Anteils Bestimmen (S1) eines Anteils von somatischen Zellen aus der biologischen Probe, die das Y-Chromosom verloren haben, umfasst.

3. Verfahren nach Anspruch 1 oder Anspruch 2, wobei die biologische Probe eine Blut-, blutabgeleitete, Lymph-, Speichel-, Urin-, Stuhl-, CSF-, Haut- oder Haarprobe ist.

4. Verfahren nach einem der Ansprüche 1 bis 3, wobei die Zellen in Schritt S1 kernhaltige Blutzellen aus einer von der männlichen Person erhaltenen Blutprobe sind, wobei Bestimmen (S1) des Anteils Bestimmen (S1) eines Anteils kernhaltiger Blutzellen aus der Blutprobe, die das Y-Chromosom verloren haben, umfasst.

5. Verfahren nach einem der Ansprüche 1 bis 4, weiter umfassend Erhalten einer Teilmenge oder eines Anteils von Zellen aus der biologischen Probe und Bestimmen (S1) eines Anteils von Zellen aus der Teilmenge oder dem Anteil, die das Y-Chromosom verloren haben.

6. Verfahren nach einem der Ansprüche 1 bis 5, wobei Bestimmen (S1) des Anteils Bestimmen eines Anteils von CD4+ T-Zellen, die das Y-Chromosom verloren haben, umfasst.

7. Verfahren nach einem der Ansprüche 1 bis 6, wobei der vordefinierte Schwellenwert im Bereich von 5 bis 20 % von Zellen liegt.

8. Verfahren nach einem der Ansprüche 1 bis 7, wobei Vergleichen (S2) des Anteils Vergleichen (S2) des Anteils mit einem vordefinierten Schwellenwert von etwa 18 % umfasst.

9. Verfahren nach einem der Ansprüche 1 bis 7, wobei Vergleichen (S2) des Anteils Vergleichen (S2) des Anteils mit einem vordefinierten Schwellenwert von etwa 10 % umfasst.

10. Verfahren nach einem der Ansprüche 1 bis 9, wobei ein vordefinierter Schwellenwert auf das untere 99 % Konfidenzintervall der Verteilung eines erwarteten experimentellen Hintergrundrauschens bei einer Bestimmung der Verlusthäufigkeit des Y-Chromosoms gesetzt wird.

11. Verfahren nach einem der Ansprüche 1 bis 10, wobei
Vergleichen (S2) des Anteils Vergleichen (S2) des Anteils mit einem ersten vordefinierten Schwellenwert und einem zweiten vordefinierten Schwellenwert, der höher als der erste vordefinierte Schwellenwert ist, umfasst und
Vorhersagen (S3), ob die männliche Person ein erhöhtes Risiko für die Entwicklung der Alzheimerkrankheit aufweist, umfasst:
Vorhersagen, dass die männliche Person kein erhöhtes Risiko für die Entwicklung der Alzheimerkrankheit aufweist, wenn der Anteil niedriger als der erste vordefinierte Schwellenwert ist;
Vorhersagen, dass die männliche Person ein erhöhtes Risiko für die Entwicklung der Alzheimerkrankheit aufweist, wenn der Anteil gleich oder größer als der erste vordefinierte Schwellenwert, aber niedriger als der zweite vordefinierte Schwellenwert ist; und
Vorhersagen, dass die männliche Person ein hohes Risiko für die Entwicklung der Alzheimerkrankheit aufweist, wenn der Anteil gleich oder größer als der zweite vordefinierte Schwellenwert ist.

12. Verfahren nach Anspruch 11, wobei Vergleichen (S2) des Anteils Vergleichen (S2) des Anteils mit dem ersten vordefinierten Schwellenwert von etwa 18 % und dem zweiten vordefinierten Schwellenwert von etwa 35 % umfasst.

13. Verfahren nach einem der Ansprüche 1 bis 12, wobei Bestimmen (S1) des Anteils umfasst Bestimmen (S1) des Anteils basierend auf;
(i) einer Einzel-Nukleotid-Polymorphismus-, SNP, Array-Analyse unter Verwendung von Datenpunkten, die von einer pseudoautosomalen Region 1, PAR1, Region der X- und Y-Chromosomen abgeleitet sind; oder
(ii) Quantifizierung der Datenpunkte, die von der PAR1-Region auf den X- und Y-Chromosomen abgeleitet sind, unter Verwendung einer Korrelation zwischen einem Log R Verhältnis, LRR, in der PAR1-Region von Y-Chromosom und einer absoluten Abweichung von einem erwarteten diploide B-Allel-Frequenz-, BAF, Wert von 0,5.

14. Verfahren nach einem der Ansprüche 1 bis 12, wobei Bestimmen (S1) des Anteils Bestimmen (S1) des Anteils basierend auf Sequenzieren einer auf Y-Chromosom vorhandenen DNA-Region umfasst.

15. Verfahren nach einem der Ansprüche 1 bis 14, wobei die Person gesund ist, nicht an einer Alzheimerkrankheit leidet oder nicht als eine Alzheimerkrankheit aufweisend identifiziert ist.

16. Verwendung des Verlusts des Y-Chromosoms in Zellen aus einer biologischen Probe, die von einer männlichen Person erhalten wurde, als einen genetischen Marker zur Vorhersage, ob die männliche Person ein erhöhtes Risiko für die Entwicklung der Alzheimerkrankheit aufweist.

## Revendications

1. Procédé de prédiction qu'un sujet masculin présente ou non un risque accru de développer la maladie d'Alzheimer, comprenant :
la détermination (S1) d'une fraction de cellules provenant d'un échantillon biologique obtenu à partir dudit sujet masculin qui ont perdu le chromosome Y ;
la comparaison (S2) de ladite fraction avec un seuil prédéfini ; et
la prédiction (S3) que ledit sujet masculin présente ou non un risque accru de développer la maladie d'Alzheimer sur la base de ladite comparaison entre ladite fraction et ledit seuil prédéfini, dans lequel :
ledit sujet masculin présente un risque accru de développer la maladie d'Alzheimer si ladite fraction est égale ou supérieure audit seuil prédéfini ; ou
ledit sujet masculin ne présente aucun risque accru de développer la maladie d'Alzheimer si ladite fraction est inférieure audit seuil prédéfini.

2. Procédé selon la revendication 1, dans lequel la détermination (S1) de ladite fraction comprend la détermination (S1) d'une fraction de cellules somatiques provenant dudit échantillon biologique qui ont perdu le chromosome Y.

3. Procédé selon la revendication 1 ou la revendication 2, dans lequel l'échantillon biologique est un échantillon de sang, dérivé du sang, de lymphe, de salive, d'urine, de selles, de liquide céphalo-rachidien (LCR), de peau ou de cheveu.

4. Procédé selon l'une quelconque des revendications 1 à 3, dans lequel les cellules dans l'étape S1 sont des cellules sanguines nucléées provenant d'un échantillon de sang obtenu auprès dudit sujet masculin, dans lequel la détermination (S1) de ladite fraction comprend la détermination (S1) d'une fraction de cellules sanguines nucléées provenant dudit échantillon de sang qui ont perdu le chromosome Y.

5. Procédé selon l'une quelconque des revendications 1 à 4, comprenant en outre l'obtention d'un sous-ensemble ou d'une fraction de cellules provenant de l'échantillon biologique et la détermination (S1) d'une fraction de cellules provenant dudit sous-ensemble ou de ladite fraction qui ont perdu le chromosome Y.

6. Procédé selon l'une quelconque des revendications 1 à 5, dans lequel la détermination (S1) de ladite fraction comprend la détermination d'une fraction de cellules T CD4+ qui ont perdu le chromosome Y.

7. Procédé selon l'une quelconque des revendications 1 à 6, dans lequel dit seuil prédéfini se situe dans la plage de 5 à 20 % de cellules.

8. Procédé selon l'une quelconque des revendications 1 à 7, dans lequel la comparaison (S2) de ladite fraction comprend la comparaison (S2) de ladite fraction avec un seuil prédéfini d'environ 18 %.

9. Procédé selon l'une quelconque des revendications 1 à 7, dans lequel la comparaison (S2) de ladite fraction comprend la comparaison (S2) de ladite fraction avec un seuil prédéfini d'environ 10 %.

10. Procédé selon l'une quelconque des revendications 1 à 9, dans lequel un seuil prédéfini est fixé dans l'intervalle de confiance inférieur à 99 % de la distribution de bruit de fond expérimental attendu dans une détermination de fréquence de perte de chromosome Y.

11. Procédé selon l'une quelconque des revendications 1 à 10, dans lequel
la comparaison (S2) de ladite fraction comprend la comparaison (S2) de ladite fraction avec un premier seuil prédéfini et avec un second seuil prédéfini qui est supérieur audit premier seuil prédéfini, et
la prédiction (S3) que ledit sujet masculin présente ou non un risque accru de développer la maladie d'Alzheimer comprend :
la prédiction que ledit sujet masculin ne présente aucun risque accru de développer la maladie d'Alzheimer si ladite fraction est inférieure audit premier seuil prédéfini ;
la prédiction que ledit sujet masculin présente un risque accru de développer la maladie d'Alzheimer si ladite fraction est supérieure ou égale audit premier seuil prédéfini mais inférieure audit second seuil prédéfini ; et la prédiction que ledit sujet masculin présente un risque élevé de développer la maladie d'Alzheimer si ladite fraction est supérieure ou égale audit second seuil prédéfini.

12. Procédé selon la revendication 11, dans lequel la comparaison (S2) de ladite fraction comprend la comparaison (S2) de ladite fraction avec ledit premier seuil prédéfini d'environ 18 % et avec ledit second seuil prédéfini d'environ 35 %.

13. Procédé selon l'une quelconque des revendications 1 à 12, dans lequel la détermination (S1) de ladite fraction comprend la détermination (S1) de ladite fraction sur la base de :
(i) une analyse de matrice de polymorphisme mononucléotidique, PSN, en utilisant des points de données dérivés d'une région pseudo-autosomique 1, PAR1, d'une région des chromosomes X et Y ; ou
(ii) une quantification desdits points de données dérivés de ladite région PAR1 sur les chromosomes X et Y en utilisant une corrélation entre un rapport de log R, LRR, dans ladite région PAR1 du chromosome Y et un écart absolu par rapport à une valeur de fréquence d'allèle B (BAF) diploïde attendue de 0,5.

14. Procédé selon l'une quelconque des revendications 1 à 12, dans lequel la détermination (S1) de ladite fraction comprend la détermination (S1) de ladite fraction sur la base du séquençage d'une région d'ADN présente sur le chromosome Y.

15. Procédé selon l'une quelconque des revendications 1 à 14, dans lequel le sujet est en bonne santé, ne souffre pas de la maladie d'Alzheimer ou n'est pas identifié comme souffrant de la maladie d'Alzheimer.

16. Utilisation de la perte de chromosome Y dans des cellules d'un échantillon biologique obtenu auprès d'un sujet masculin en tant que marqueur génétique pour prédire si ledit sujet masculin présente ou non un risque accru de développer la maladie d'Alzheimer.
